# EUROPÄISCHE PATENTSCHRIFT

(11) **EP 3 817 676 B1**
(45) Veröffentlichungstag und Bekanntmachung des Hinweises auf die Patenterteilung: **01.11.2023**
(21) Anmeldenummer: 19739230.1
(22) Anmeldetag: 04.07.2019
(51) Int. Cl.: A61B 17/70

(54) **VORRICHTUNG ZUR KORREKTUR EINER KNOCHENFRAKTUR**
DEVICE FOR CORRECTING BONE FRACTURES
DISPOSITIF DE CORRECTION D'UNE FRACTURE OSSEUSE

(30) Priorität: 04.07.2018 DE 102018116177
(43) Veröffentlichungstag der Anmeldung: 12.05.2021
(73) Patentinhaber: Silony Medical International AG, 8500 Frauenfeld (CH)
(72) Erfinder: HEUER, Frank, 70794 Filderstadt (DE)
(74) Vertreter: DREISS Patentanwälte PartG mbB
(86) Internationale Anmeldenummer: PCT/EP2019/067967
(87) Internationale Veröffentlichungsnummer: WO 2020/007968

(56) Entgegenhaltungen:
- DE-B3-102016 121 054
- US-A1- 2011 077 689
- US-A1- 2012 191 143
- US-B1- 9 907 582

## Beschreibung

Die vorliegende Erfindung betrifft eine Vorrichtung zur Korrektur einer Knochenfraktur, wobei die Vorrichtung dazu ausgebildet ist, dass die Korrektur der Knochenfraktur unter Verwendung von Knochenankern, insbesondere Knochenschrauben, insbesondere monoaxialen Knochenschrauben oder polyaxialen Knochenschrauben, die in ihrer Polyaxailität temporär blockiert werden können, und an den Knochenschrauben anbringbaren Verlängerungsvorrichtungen durchgeführt wird.

Eine Polyaxialschraube ist beispielsweise aus der US 6,063,090 bekannt. Bei Polyaxialschrauben ist ein Gabelkopf gegenüber einem Schraubanker verschwenkbar angebracht. Über entsprechende Vorrichtungen lässt sich der Schraubanker gegenüber dem Gabelkopf in seiner Position festlegen. Eine monoaxiale Pedikelschraube besitzt einen Schraubanker, welcher in Verlängerung in proximaler Richtung einen Gabelkopf besitzt. Die erfindungsgemäße Vorrichtung eignet sich insbesondere zur Verwendung mit einem Knochenanker, der ausgebildet ist, um eine ortsfeste und orientierungsfixierte Verbindung zu einem Knochensegment herzustellen. Dies kann bspw. mit monoaxialen Knochenankern oder mit beweglichen (polyaxialen) Knochenankern, die jedoch in einer bestimmten Orientierung fixiert werden können, realisiert werden.

Eine Verlängerungsvorrichtung im vorliegenden Sinne ist eine entlang einer Richtung einer Längserstreckung länglich ausgebildete Vorrichtung, die an die Knochenschraube, beispielsweise am Gabelkopf, angebracht wird und mittels derer ein Hebel zum Verschwenken der Knochenschraube bzw. des Gabelkopfes bereitgestellt wird. Verlängerungsvorrichtungen in diesem Sinne können auch Teil des erfindungsgemäßen und später noch erläuterten Systems sein. Die Verlängerungsvorrichtung dient jedoch üblicherweise nicht nur als Hebelarm, sondern auch um beispielsweise einen Korrekturstab in den Gabelkopf einzusetzen. Insbesondere kann/können die Verlängerungsvorrichtung(en) rohrartig, insbesondere entlang ihrer Längserstreckung seitlich einfach oder an mehreren um den Umfang verteilten Stellen seitlich geschlitzt ausgebildet sein. Bspw. können sich ein oder mehrere seitliche Schlitze von dem zur Verbindung mit der Knochenschraube vorgesehenen Ende entlang der Längserstreckung erstrecken, wobei die Schlitze sich vorzugsweise nicht über die gesamte Längserstreckung erstrecken. Denkbar ist jedoch, dass sich beispielsweise ein Schlitz über die gesamte Längserstreckung erstreckt. Eine Verlängerungsvorrichtung kann jedoch auch zwei voneinander unabhängige "halbschalenartige" Elemente umfassen. Vorzugsweise ist die Verlängerungsvorrichtung jedoch im Wesentlichen röhrenartig ausgebildet. Vorrichtungen zur Korrektur einer Knochenfraktur sind unter anderem aus US 9 907 582 B1, US 2012/191143 A1, US 2011/077689 A1 und DE 10 2016 121054 B3 bekannt.

Aufgabe der vorliegenden Erfindung ist es, eine Vorrichtung bereitzustellen die geeignet ist, um in kontrollierter Weise Knochenschrauben mit daran angebrachten Verlängerungsvorrichtungen zu manipulieren, wobei sich die Vorrichtung möglichst vorteilhaft an den Verlängerungsvorrichtungen anbringen lässt.

Diese Aufgabe wird erfindungsgemäß durch eine Vorrichtung nach Anspruch 1 gelöst. Teil der Erfindung ist auch ein System aus einer erfindungsgemäßen Vorrichtung sowie zwei Verlängerungsvorrichtungen, insbesondere und zwei Knochenankern, insbesondere Polyaxialschrauben. Vorteilhafte Weiterbildungen der Erfindung sind Gegenstand der abhängigen Ansprüche.

Die Vorrichtung umfasst eine erste Aufnahme und eine zweite Aufnahme. Die erste Aufnahme ist dazu ausgebildet und angeordnet, eine erste Verlängerungsvorrichtung für eine erste Knochenschraube aufzunehmen. Die erste Aufnahme ist auch dazu ausgebildet und angeordnet einen ersten Krafteinleitungsabschnitt in die erste Verlängerungsvorrichtung zu bilden. Über die erste Aufnahme können Kräfte, über die die Stellung der ersten Verlängerungsvorrichtung beeinflussbar ist, in die Verlängerungsvorrichtung eingeleitet werden. Bspw. kann die Verlängerungsvorrichtung durch diese Krafteinleitung geschwenkt werden. Die zweite Aufnahme wiederum ist dazu ausgebildet und angeordnet, eine zweite Verlängerungsvorrichtung für eine zweite Knochenschraube aufzunehmen. Die zweite Aufnahme ist auch dazu ausgebildet und angeordnet einen zweiten Krafteinleitungsabschnitt in die zweite Verlängerungsvorrichtung zu bilden. Über die zweite Aufnahme können Kräfte, über die die Stellung der zweiten Verlängerungsvorrichtung beeinflussbar ist, in die Verlängerungsvorrichtung eingeleitet werden. Bspw. kann die Verlängerungsvorrichtung durch diese Krafteinleitung geschwenkt werden.

Die Vorrichtung umfasst eine erste Betätigungseinrichtung und eine zweite Betätigungseinrichtung. Die Vorrichtung ist derart ausgebildet, dass die erste Verlängerungsvorrichtung eine erste obere vorgesehene Schwenkachse und eine zu dieser beabstandete erste untere vorgesehene Schwenkachse aufweist und die zweite Verlängerungsvorrichtung eine zweite obere vorgesehene Schwenkachse und eine zu dieser beabstandete zweite untere vorgesehene Schwenkachse aufweist, wenn die Verlängerungsvorrichtungen in den vorgesehenen Positionen in den Aufnahmen aufgenommen sind.

Die erste Betätigungseinrichtung ist dabei ausgebildet, um bei Betätigungen die erste Verlängerungsvorrichtung und die zweite Verlängerungsvorrichtung derart zueinander zu bewegen, dass sich ein Abstand zwischen der ersten unteren Schwenkachse und der zweiten unteren Schwenkachse verändert. Dabei kann es insbesondere vorgesehen sein, dass die Änderung des Abstands durch ein Verschwenken der ersten Verlängerungsvorrichtung um die erste obere Schwenkachse und der zweiten Verlängerungsvorrichtung um die zweite obere Schwenkachse bewirkt wird. Mit anderen Worten, bei Betätigung der ersten Betätigungseinrichtung schwenken beide Verlängerungsvorrichtungen um ihre jeweiligen oberen Schwenkachsen. Bei alleiniger Betätigung der ersten Betätigungseinrichtung bleibt dabei der Abstand zwischen den oberen Schwenkachsen gleich und lediglich der Abstand zwischen den unteren Schwenkachsen ändert sich.

Die zweite Betätigungseinrichtung ist ausgebildet, um bei deren Betätigung die erste Verlängerungsvorrichtung um die erste untere Schwenkachse und die zweite Verlängerungsvorrichtung um die zweite untere Schwenkachse zu verschwenken. Mit anderen Worten, bei Betätigung der zweiten Betätigungseinrichtung schwenken beide Verlängerungsvorrichtungen um ihre jeweiligen unteren Schwenkachsen. Bei alleiniger Betätigung der zweiten Betätigungseinrichtung bleibt dabei der Abstand zwischen den unteren Schwenkachsen gleich und lediglich der Abstand zwischen den oberen Schwenkachsen ändert sich.

Der erste Krafteinleitungsabschnitt ist zu einer der ersten Schwenkachsen, insbesondere zu der ersten unteren Schwenkachse, in Richtung der Längserstreckung der ersten Verlängerungsvorrichtung (bezogen auf deren vorgesehene Position, wenn sie in der Aufnahme aufgenommen ist) beabstandet und der zweite Krafteinleitungsabschnitt ist zu einer der zweiten Schwenkachsen, insbesondere zu der zweiten unteren Schwenkachse, in Richtung der Längserstreckung der zweiten Verlängerungsvorrichtung beabstandet, wenn die Verlängerungsvorrichtungen in den vorgesehenen Positionen in den Aufnahmen aufgenommen sind. Mit anderen Worten, beim Blick in Richtung der Längserstreckung der jeweiligen Verlängerungsvorrichtung liegen der jeweilige Krafteinleitungsabschnitt und eine der jeweiligen Schwenkachsen, insbesondere die untere Schwenkachse, hintereinander in einem Abstand zueinander. Nochmals anders ausgedrückt, die Aufnahmen befinden sich entlang der Längserstreckung der Verlängerungsvorrichtungen gesehen an einer anderen Stelle der Verlängerungsvorrichtung als eine der Schwenkachsen, vorzugsweise sind die Aufnahmen beabstandet zu der unteren Schwenkachse angeordnet. Vorzugsweise erstreckt sich die obere Schwenkachse durch die Aufnahmen hindurch und die untere Schwenkachse ist zu den Aufnahmen beabstandet. Vorzugsweise werden die Verlängerungsvorrichtungen im Bereich der unteren Schwenkachsen von keinem Teil der Vorrichtung kontaktiert.

Hierdurch kann die Vorrichtung besonders einfach und komfortabel an den Verlängerungsvorrichtungen angebracht werden. Insbesondere, wenn die Krafteinleitungsabschnitte von den unteren Schwenkachsen beabstandet sind, bietet dies eine besonders gute Zugänglichkeit der Operationsstelle für den Operateur. Die gesamte Vorrichtung kann zur Operationsstelle, die typischer weise sehr nah an den unteren Schwenkachsen liegt, beabstandet angeordnet sein.

Die Vorrichtung lässt sich auch wie folgt beschreiben:
Vorrichtung zur Korrektur einer Knochenfraktur unter Verwendung von Knochenankern, insbesondere Knochenschrauben, und an den Knochenankern anbringbaren Verlängerungsvorrichtungen,
wobei die Vorrichtung eine erste Aufnahme und eine zweite Aufnahme umfasst,
wobei die erste Aufnahme dazu ausgebildet und angeordnet ist, eine erste Verlängerungsvorrichtung für einen ersten Knochenanker in einer vorgesehenen Position in der Aufnahme aufzunehmen und einen ersten Krafteinleitungsabschnitt in die erste Verlängerungsvorrichtung zu bilden, und
wobei die zweite Aufnahme dazu ausgebildet und angeordnet ist, eine zweite Verlängerungsvorrichtung für einen zweiten Knochenanker in einer vorgesehenen Position in der Aufnahme aufzunehmen und einen zweiten Krafteinleitungsabschnitt in die zweite Verlängerungsvorrichtung zu bilden,
wobei die Vorrichtung eine erste Betätigungseinrichtung und eine zweite Betätigungseinrichtung umfasst,
wobei die Vorrichtung derart ausgebildet ist, dass die erste Verlängerungsvorrichtung in der vorgesehenen Position eine erste obere vorgesehene Schwenkachse und eine zu dieser beabstandete erste untere vorgesehene Schwenkachse aufweist und die zweite Verlängerungsvorrichtung in der vorgesehenen Position eine zweite obere vorgesehene Schwenkachse und eine zu dieser beabstandete zweite untere vorgesehene Schwenkachse aufweist, wenn die Verlängerungsvorrichtungen in den vorgesehenen Positionen in den Aufnahmen aufgenommen sind,
wobei die erste Betätigungseinrichtung ausgebildet ist, um bei Betätigung die erste Verlängerungsvorrichtung und die zweite Verlängerungsvorrichtung derart zueinander zu bewegen, dass sich ein Abstand zwischen der ersten unteren Schwenkachse und der zweiten unteren Schwenkachse verändert, insbesondere wobei die Änderung des Abstands durch ein Verschwenken der ersten Verlängerungsvorrichtung um die erste obere Schwenkachse und der zweiten Verlängerungsvorrichtung um die zweite obere Schwenkachse bewirkt wird, und
wobei die zweite Betätigungseinrichtung ausgebildet ist, um bei deren Betätigung die erste Verlängerungsvorrichtung um die erste untere Schwenkachse und die zweite Verlängerungsvorrichtung um die zweite untere Schwenkachse zu verschwenken,
dadurch gekennzeichnet, dass
der durch die erste Aufnahme gebildete erste Krafteinleitungsabschnitt in Richtung der Längserstreckung der ersten Verlängerungsvorrichtung zu einer der ersten Schwenkachsen, vorzugsweise der ersten unteren Schwenkachse, beabstandet ist und der durch die zweite Aufnahme gebildete zweite Krafteinleitungsabschnitt in Richtung der Längserstreckung der zweiten Verlängerungsvorrichtung zu einer der zweiten Schwenkachsen, vorzugsweise der zweiten unteren Schwenkachse, beabstandet ist, wenn die Verlängerungsvorrichtungen in den vorgesehenen Positionen in den Aufnahmen aufgenommen sind und dass die unteren Schwenkachsen derart angeordnet sind, dass sie gegenüber den Verlängerungsvorrichtungen in einer Verlängerung der Längserstreckung dieser Verlängerungsvorrichtungen beabstandet zu diesen angeordnet sind. Entsprechend ist die Vorrichtung ausgebildet, um mit den Verlängerungsvorrichtungen ein System, wie es später noch im Detail erörtert wird, zu bilden.

Nachfolgend wird auf die Einzelnen Aspekte der Vorrichtung und auf deren mögliche Ausgestaltung eingegangen sowie weitere mögliche Ausführungsformen der Vorrichtung beschrieben.

Die Aufnahmen dienen dazu die Verlängerungsvorrichtungen zu erfassen und die zur Bewegung bzw. zum Verschwenken der Verlängerungsvorrichtungen benötigten Kräfte in diese einzuleiten, da die Aufnahmen die Krafteinleitungsabschnitte bilden.

Die Aufnahmen können beispielsweise klammerartig ausgebildet sein. Die Aufnahmen können insbesondere zum lösbaren, insbesondere umgreifenden, klemmenden Befestigen an einem mittels der Aufnahmen teilweise umgreifbaren Außenumfang der insbesondere stab- oder rohrförmigen Verlängerungsvorrichtungen ausgebildet sein.

Der durch erste Aufnahme gebildete erste Krafteinleitungsabschnitt und der durch die zweite Aufnahme gebildete zweite Krafteinleitungsabschnitt können die einzigen Krafteinleitungsabschnitte in die Verlängerungsvorrichtungen sein. Mit anderen Worten ist dann vorgesehen, dass Kräfte zur Bewegung, insbesondere zum Verschwenken der Verlängerungsvorrichtungen, durch die Vorrichtung ausschließlich über diese Krafteinleitungsabschnitte in die Verlängerungsvorrichtungen einleitbar sind.

Die Schwenkachsen (oberen und unteren) können orthogonal zur Längsrichtung bzw. zur Längserstreckung der Verlängerungsvorrichtungen verlaufen.

Insbesondere können die Aufnahmen mit einem entlang einer Umfangs- und Längsrichtung der Verlängerungsvorrichtung erstreckten, nachgiebig auslenkbaren Schenkel ausgebildet sein, welcher von außen an den Außenumfang der Verlängerungsvorrichtung angelegt werden kann, wobei der wenigstens eine Schenkel eine Außenwand und eine Innenwand aufweist, die an wenigstens einer Stelle so miteinander verbunden sind, dass beim Gegeneinanderziehen von Außenwand und Innenwand in radialer Richtung der Verlängerungsvorrichtung an einer Zugeinleitungsstelle mittels eines Zugeinleitungsmechanismus eine ausgehend von der Zugeinleitungsstelle in der Umfangsrichtung der Verlängerungsvorrichtung wirkende Druckspannung in der Außenwand und eine in entgegengesetzter Umfangsrichtung wirkende Zugspannung in der Innenwand erzeugt wird, wodurch der Schenkel gegen den Außenumfang der Verlängerungsvorrichtung gezwungen wird und dadurch die Aufnahme an der Verlängerungsvorrichtung klemmend anordenbar ist bzw. die Verlängerungsvorrichtung durch die Aufnahme klemmend greifbar ist. Dies stellt eine besonders sichere und komfortable Ausbildung der Aufnahmen dar. Es kann auch lediglich eine der Aufnahmen wie eben oder nachfolgend beschrieben ausgebildet sein.

Vorzugsweise sind die Aufnahmen (oder eine Aufnahme) derart ausgebildet, dass beidseitig von der Zugeinleitungsstelle jeweils ein in der Umfangsrichtung und in der Längsrichtung der Verlängerungsvorrichtung erstreckter nachgiebig auslenkbarer Schenkel vorgesehen ist, welcher von außen an den Außenumfang der Verlängerungsvorrichtung angelegt werden kann, wobei jeder Schenkel eine Außenwandung und eine Innenwandung aufweist, die an wenigstens einer Stelle so miteinander verbunden sind, dass beim Gegeneinanderziehen von Außenwandung und Innenwandung in radialer Richtung der Verlängerungsvorrichtung an der Zugeinleitungsstelle mittels eines Zugeinleitungsmechanismus eine ausgehend von der Zugeinleitungsstelle in der Umfangsrichtung der Verlängerungsvorrichtung wirkende Druckspannung in der Außenwand und eine in entgegengesetzter Umfangsrichtung wirkende Zugspannung in der Innenwand des jeweiligen Schenkels erzeugt wird, wodurch der jeweilige Schenkel gegen den Außenumfang der Verlängerungsvorrichtung gezwungen wird und dadurch die Aufnahme an der Verlängerungsvorrichtung klemmend anordenbar ist.

Vorzugsweise sind die Aufnahmen (oder eine Aufnahme) derart ausgebildet, dass die Außenwand und die Innenwand zumindest im Bereich eines in Umfangsrichtung freien Endes des Schenkels bzw. der Schenkel miteinander verbunden sind. Insbesondere können die Außenwand und die Innenwand im Bereich des in Umfangsrichtung freien Endes des Schenkels bzw. der Schenkel einstückig ineinander übergehen. Denkbar im Sinne der Erfindung ist auch, dass die Außenwand und die Innenwand der Aufnahme weiter durch zusätzliche Streben oder Stege miteinander verbunden sind, die in unbelastetem Anfangszustand der Aufnahme, vorzugsweise im Wesentlichen in einer radialen Ebene erstreckt sind. Die Streben oder Stege können einstückig in die Außenwand und/oder in die Innenwand übergehen.

Der Zugeinleitungsmechanismus der Aufnahme kann insbesondere derart ausgeführt sein, dass im Bereich der Zugeinleitungsstelle die Innenwand in Richtung der Außenwand bewegbar bzw. ziehbar ist. Hierzu kann der Zugeinleitungsmechanismus insbesondere eine Exzenterstellvorrichtung oder eine Kniehebelstellvorrichtung oder ein Parallelogrammgetriebe umfassen.

Andere Arten von Aufnahmen, bspw. über einen Schraubmechanismus verspannbare Klammern können ebenso verwendet werden.

Die unteren und oberen Schwenkachsen der Verlängerungsvorrichtungen sind entlang der Längsrichtung der Verlängerungsvorrichtung zueinander beabstandet. Über ein Verschwenken um die untere oder obere Schwenkachse kann die Winkelstellung der Verlängerungsvorrichtung und damit die Winkelstellung der Knochenschraube verändert werden. Ein gleichzeitiges Verschwenken um die obere und untere Schwenkachse beider Verlängerungsvorrichtungen kann dazu genutzt werden, um eine translatorische Bewegung der Verlängerungsvorrichtungen zueinander zu bewirken. Insbesondere ist es möglich, die Verlängerungsvorrichtungen hierdurch ohne ein Verändern der Winkelstellung der Verlängerungsvorrichtung zueinander translatorisch zu bewegen. Das überlagerte Verschwenken um beide Schwenkachsen kann also quasi in einer rein translatorischen Bewegung resultieren. Mit der Vorrichtung kann also beispielsweise der Abstand zwischen den beiden Knochenankern eingestellt werden und anschließend können die Knochenanker um die jeweiligen Schwenkachsen geschwenkt werden.

Insbesondere kann also mittels der Vorrichtung eine Parallelverschiebung der Verlängerungsvorrichtungen zueinander erreicht werden. Eine derartige

Parallelverschiebung eignet sich bspw. zum Distrahieren von Knochensegmenten um eine Fraktur herum.

Beispielsweise kann die erste Knochenschraube, an der die erste Verlängerungsvorrichtung angebracht ist, in einen ersten intakten Wirbelknochen der Wirkbelsäule eingesetzt werden und die zweite Knochenschraube mit der daran angebrachten Verlängerungsvorrichtung in einen zweiten Wirbelknochen der Wirbelsäule, wobei zwischen dem ersten und zweiten Wirbelknochen der Wirbelsäule ein frakturierter Wirbelknochen angeordnet ist.

Durch eine Parallelverschiebung der Verlängerungsvorrichtungen und den daran angebrachten Knochenschrauben mittels der erfindungsgemäßen Vorrichtung kann der frakturierte Wirkbelknochen entlastet und anschließend behandelt werden.

Durch ein Verschwenken der Verlängerungsvorrichtung kann die Anordnung des ersten und zweiten Wirbelknochens zueinander moduliert bspw. korrigiert werden.

Ein entsprechendes Verfahren unter Verwendung der erfindungsgemäßen Vorrichtung wird ebenfalls beschrieben, das keinen Teil der beanspruchten Erfindung bildet. Es dient lediglich dem Verständnis der Erfindung.

Das Verfahren umfasst die Schritte:
Verbinden einer ersten Verlängerungsvorrichtung mit dem ersten Knochenanker;
Einbringen eines ersten Knochenankers in einen ersten Knochen oder ersten Knochenabschnitt;
Verbinden einer zweiten Verlängerungsvorrichtung mit dem zweiten Knochenanker;
Einbringen eines zweiten Knochenankers in einen zweiten Knochen oder zweiten Knochenabschnitt;
Anbringen einer erfindungsgemäßen Vorrichtung an den Verlängerungsvorrichtungen, durch Aufnahme der Verlängerungsvorrichtungen in den entsprechenden Aufnahmen;
Manipulieren der Stellung des ersten Knochens oder ersten Knochenabschnitts und des zweiten Knochens oder zweiten Knochenabschnitts zueinander unter Verwendung der erfindungsgemäßen Vorrichtung.

Vorzugsweise umfasst das eben genannte Manipulieren der Stellung zunächst ein Manipulieren des Abstandes des ersten Knochens oder ersten Knochenabschnitts und des zweiten Knochens oder zweiten Knochenabschnitts zueinander unter Verwendung der erfindungsgemäßen Vorrichtung. Ein derartiges Manipulieren des Abstandes kann bspw. durch die Betätigung der ersten Betätigungseinrichtung erreicht werden, was das Verschwenken der Verlängerungseinrichtung um die oberen Schwenkachsen bewirkt und damit eine Abstandsänderung der unteren Schwenkachsen erzeugt. Ein Manipulieren des Abstandes kann auch durch Betätigung beider Betätigungseinrichtungen erreicht werden. Vorzugsweise werden beide Betätigungseinrichtungen derart synchron miteinander betätigt, dass die Abstandsänderung der unteren Schwenkachsen durch eine Parallelverschiebung (gleiche Änderung der Abstände der oberen und unteren Schwenkachsen) der Verlängerungsvorrichtungen bzw. der mit ihnen verbundenen Knochenanker. Die Abstandsänderung der unteren Schwenkachsen wird über die Knochenanker auf die Knochensegmente übertragen.

Damit kann der Abstand der Knochensegmente definiert eingestellt und konstant gehalten werden. Bei der Behandlung einer Fraktur kann damit bspw. weiterer Schaden am umliegenden Gewebe verhindert werden.

Vorzugsweise umfasst das oben genannte Manipulieren der Stellung anschließend an das o.g. Manipulieren des Abstandes ein Manipulieren der Schwenkstellung der Knochen bzw. Knochenabschnitte zueinander, wobei das Manipulieren der Schwenkstellung vorzugsweise unter Einhaltung des konstanten Abstandes der unteren Schwenkachsen zueinander erfolgt. das Manipulieren Schwenkstellung erfolgt über die Betätigung der zweiten Betätigungseinrichtung. Wird dabei die erste Betätigungseinrichtung nicht betätigt, so bleibt der Abstand der unteren Schwenkachsen zueinander konstant und die Verlängerungsvorrichtungen bzw. der mit ihnen jeweils verbundene Knochenanker verschwenkt um die jeweilige untere Schwenkachsen.

Nach der Einstellung des Abstands und/oder der Orientierung der Knochensegmente mit Hilfe der erfindungsgemäßen Vorrichtung, können die Knochenanker mit einem Verbindungselement (vorzugsweise einen stabförmigen Element) miteinander verbunden und mit Hilfe von Verbindungsmitteln (z.B. Madenschrauben) in ihrer relativen Position und Ausrichtung zueinander fixiert werden.

Vorzugsweise kann anschließend die erfindungsgemäße Vorrichtung von den Verlängerungsvorrichtungen entfernt werden.

Vorzugsweise können abschließend die Verlängerungsvorrichtungen von den Knochenankern entkoppelt werden.

Die erfindungsgemäße Vorrichtung kann derart ausgebildet sein, dass die erste Betätigungseinrichtung ausgebildet ist, um die Schwenkbewegung um die Abstandsänderung zwischen den unteren Schwenkachsen bzw. die Schwenkbewegung um die oberen Schwenkachsen durch eine Längenänderung eines längenveränderbaren Abschnitts eines ersten Stellelements, insbesondere eines Abschnitts einer ersten Gewindestange, zu bewirken. Alternativ oder zusätzlich kann die zweite Betätigungseinrichtung ausgebildet sein, um die Schwenkbewegung um die unteren Schwenkachsen durch eine Längenänderungen eines längenveränderbaren Abschnitts eines zweiten Stellelements, insbesondere eines Abschnitts einer zweiten Gewindestange, zu bewirken. Dies stellt eine einfache Möglichkeit der Ausbildung der Betätigungseinrichtungen dar.

Bei der erfindungsgemäßen Vorrichtung kann vorgesehen sein, dass das erste Stellelement in einer der ersten Verlängerungsvorrichtungen zugeordneten Führung und in einer zweiten Verlängerungsvorrichtung zugeordneten Führung gelagert ist. Dabei kann sich der längenveränderbare Abschnitt des ersten Stellelements von der der ersten Verlängerungsvorrichtung zugeordneten Führung bis zu der der zweiten Verlängerungsvorrichtung zugeordneten Führung erstrecken. Durch Veränderung der Länge des längenveränderbaren Abschnitts können die Führungen bzgl. einander bewegt werden, welche wiederum entsprechend mit den Aufnahmen gekoppelt sein können.

Vorzugsweise sind die Führungen derart ausgebildet, dass die längenveränderbaren Abschnitte des ersten Stellelements verschiebbar in wenigstens einer der Führungen angeordnet sind. Vorzugsweise umfasst das erste Stellelement einen Gewindeabschnitt, der in einer der Führungen in einem führungsseitigen Gewinde gelagert ist. Vorzugsweise umfasst eine der Führungen, insbesondere die Führung mit dem Gegengewinde einen Freigangsmechanismus, mittels dem der Eingriff des führungsseitigen Gewindes in das stellelementseitige Gewinde freigegeben werden kann, so dass das erste Stellelement ohne Verschrauben in dem Gewinde in der Führung verschiebbar ist. Vorzugsweise ist das Stellelement in der anderen, nicht gewindetragenden Führung, rein rotatorisch bewegbar (also translatorisch gegenüber der Führung fixiert) gelagert.

Bei der erfindungsgemäßen Vorrichtung kann insbesondere vorgesehen sein, dass das zweite Stellelement in der ersten Verlängerungsvorrichtung zugeordneten weiteren Führung und einer in der zweiten Verlängerungsvorrichtung zugeordneten weiteren Führung gelagert ist und sich der längenverändbare Abschnitt des zweiten Stellelements von der der ersten Verlängerungsvorrichtung zugeordneten weiteren Führung bis zu der der zweiten Verlängerungsvorrichtung zugeordneten weiteren Führung erstreckt.

Vorzugsweise sind die weiteren Führungen derart ausgebildet, dass die längenveränderbaren Abschnitte des zweiten Stellelements verschiebbar in wenigstens einer der weiteren Führungen angeordnet sind. Vorzugsweise umfasst das zweite Stellelement einen Gewindeabschnitt, der in einer der weiteren Führungen in einem führungsseitigen Gewinde gelagert ist. Vorzugsweise umfasst eine der weiteren Führungen, insbesondere die weitere Führung mit dem Gegengewinde einen Freigangsmechanismus, mittels dem der Eingriff des führungsseitigen Gewindes in das stellelementseitige Gewinde freigegeben werden kann, so dass das zweite Stellelement ohne Verschrauben in dem Gewinde in der weiteren Führung verschiebbar ist. Vorzugsweise ist das Stellelement in der anderen, nicht gewindetragenden weiteren Führung, rein rotatorisch bewegbar (also translatorisch gegenüber der Führung fixiert) gelagert.

Bei der erfindungsgemäßen Vorrichtung kann insbesondere vorgesehen sein, dass die der ersten Verlängerungsvorrichtung zugeordnete Führung um die erste Aufnahme schwenkbar ist und dass die der zweiten Verlängerungsvorrichtung zugeordnete Führung um die zweite Aufnahme schwenkbar ist.

Bei der erfindungsgemäßen Vorrichtung kann auch vorgesehen sein, dass eine Führung (bspw. die erste) zu der entsprechenden (also der ersten) Aufnahme in einer zur entsprechenden (also der ersten) oberen Schwenkachse orthogonalen Ebene beabstandet angeordnet ist. Es kann weiter vorgesehen sein, dass die Führung über ein entsprechendes Verbindungselement mit der Aufnahme verbunden ist. Insbesondere kann das Verbindungselement in sich starr ausgebildet sein. Insbesondere kann das Verbindungselement gegenüber der entsprechenden Aufnahme und gegenüber der entsprechenden Führung schwenkbar sein. Insbesondere kann das Verbindungselement länglich erstreckt und orthogonal verlaufend zur entsprechenden oberen Schwenkachse ausgebildet sein. Insbesondere kann vorgesehen sein dass beide Führungen mit den jeweiligen Aufnahmen auf diese Art beabstandet und verbunden sind. Es kann vorgesehen sein, dass also die der ersten Verlängerungsvorrichtung zugeordnete Führung zu der ersten Aufnahme beabstandet angeordnet ist, insbesondere und über ein in sich starres, gegenüber der Aufnahme und gegenüber der Führung schwenkbares erstes Verbindungselement mit dieser verbunden ist, und dass die der zweiten Verlängerungsvorrichtung zugeordnete Führung zu der zweiten Aufnahme beabstandet angeordnet ist, insbesondere und über ein in sich starres, gegenüber der Aufnahme und gegenüber der Führung schwenkbares zweites Verbindungselement mit dieser verbunden ist.

Insbesondere in Kombination mit dem Verbindungselement kann bei der erfindungsgemäßen Vorrichtung vorgesehen sein, dass ein aufnahmeseitiger Arm drehfest gegenüber einer der Aufnahmen angeordnet ist und sich von der Aufnahme zu einem von der Aufnahme beabstandeten und schwenkbar mit dem Arm verbundenen Kopplungselement erstreckt, welches wiederum schwenkbar mit einem führungsseitigen Arm verbunden ist, der sich wiederum von der schwenkbaren Verbindung mit dem Kopplungselement zu der Führung erstreckt und drehfest gegenüber dieser angeordnet ist. Auf diese Weise kann die erste Aufnahme der ersten Führung verbunden sein und/oder die zweite Aufnahme mit der zweiten Führung.

Die drehfeste Verbindung zwischen der Aufnahme und dem aufnahmeseitigen Arm und/oder die drehfeste Verbindung zwischen der Führung und dem führungsseitigen Arm kann bzw. können temporär lösbar sein, so dass die Winkelstellung zwischen der Aufnahme und dem aufnahmeseitigen Arm bzw. zwischen der Führung und dem führungsseitigen Arm frei schwenkbar ist. Dies kann beispielsweise durch eine Verzahnung jeweils auf den einander zugewandten Seiten des aufnahmeseitigen Arms und der Aufnahme bzw. des führungsseitigen Arms und der Führung realisiert sein. Dabei kann ein Verspannmechanismus vorgesehen sein, der die Verzahnungen ineinander ziehen kann und bei dessen Lösen die Verzahnungen voneinander gelöst werden können, sodass die beiden Teile frei zueinander bewegbar bzw. schwenkbar sind. Andere lösbare drehfeste Verbindungen sind denkbar.

Es kann also bspw. ein erster aufnahmeseitiger Arm drehfest gegenüber der ersten Aufnahme angeordnet sein und sich von der ersten Aufnahme zu einem von der ersten Aufnahme beabstandeten und schwenkbar mit dem Arm verbundenen ersten Kopplungselement erstrecken, welches wiederum schwenkbar mit einem ersten führungsseitigen Arm verbunden ist, der sich wiederum von der schwenkbaren Verbindung mit dem ersten Kopplungselement zu der ersten Führung erstreckt und drehfest gegenüber dieser angeordnet ist. Hierdurch kann eine effiziente Kopplung zwischen der Verschiebung der Führungen und über die Stellelemente und der Winkelstellung der Aufnahmen erreicht werden. Entsprechendes gilt für die Verbindung zwischen der zweiten Aufnahme der zweiten Führung.

Es kann vorgesehen sein, dass das Verbindungselement, das Kopplungselement, der aufnahmeseitige Arm und der führungsseitige Arm im Wesentlichen in einer gemeinsamen Ebene angeordnet sind, insbesondere die orthogonal zu der oberen Schwenkachse verläuft. Mit in einer Ebene ist dabei auch gemeint, dass die einzelnen Teile aufeinander aufliegen, wie dies bspw. bei den nachfolgenden Figuren zu sehen ist, dass jedoch die einzelnen Teile in dieser Ebene erstreckt sind und sich nicht in Richtung der Schwenkachse aus der Ebene hinaus erstrecken.

Es kann auch vorgesehen sein, dass eine Führung drehfest mit einem führungsseitigen Verzahnungsabschnitt verbunden ist. Die entsprechende Aufnahme kann drehfest mit einem aufnahmeseitigen Verzahnungsabschnitt verbunden sein.

Insbesondere können der führungsseitige Verzahnungsabschnitt und der aufnahmeseitige Verzahnungsabschnitt derart bewegungsgekoppelt sein, dass der führungsseitige Verzahnungsabschnitt und der aufnahmeseitige Verzahnungsabschnitt gleichläufige Drehrichtungen aufweisen. Insbesondere können die beiden Verzahnungsabschnitte über ein zwischen dem führungsseitigen Verzahnungsabschnitt und dem aufnahmeseitigen Verzahnungsabschnitt angeordnetes Zahnrad, insbesondere das mit dem führungsseitigen Verzahnungsabschnitt und dem aufnahmeseitigen Verzahnungsabschnitt in Eingriff steht, derart bewegungsgekoppelt sein, dass der führungsseitige Verzahnungsabschnitt und der aufnahmeseitige Verzahnungsabschnitt gleichläufige Drehrichtungen aufweisen. Beide Führungen können mit den jeweiligen Aufnahmen auf diese Art verbunden sein. Es kann vorgesehen sein, dass der führungsseitige Verzahnungsabschnitt und der aufnahmeseitige Verzahnungsabschnitt, insbesondere und das Zahnrad im Wesentlichen in einer gemeinsamen Ebene angeordnet sind, insbesondere die orthogonal zu der oberen Schwenkachse verläuft.

Die jeweilige Führung kann mit dem führungsseitigen Verzahnungsabschnitt über ein in sich starres, gegenüber der entsprechenden Aufnahme und gegenüber der Führung schwenkbares Verbindungselement mit der entsprechenden Aufnahme verbunden sein. Das Verbindungselement kann länglich erstreckt ausgebildet und orthogonal zur entsprechenden oberen Schwenkachse angeordnet sein. An dem Verbindungselement kann das Zahnrad drehbar gelagert angeordnet sein.

Die drehfeste Verbindung zwischen der Aufnahme und dem aufnahmeseitigen Verzahnungsabschnitt kann temporär lösbar sein. Ebenso oder alternativ kann die drehfeste Verbindung zwischen der Führung und dem führungsseitigen Verzahnungsabschnitt temporär lösbar sein. Entsprechend kann dann die Winkelstellung zwischen der Aufnahme und dem aufnahmeseitigen Verzahnungsabschnitt bzw. zwischen der Führung und dem führungsseitigen Verzahnungsabschnitt frei schwenkbar sein. Hierdurch kann beispielsweise der Abdeckbereiche Winkelbereich der Verschränkung der Verlängerungsvorrichtung zueinander erhöht werden.

Im Sinne der vorliegenden Erfindung ist insbesondere, wenn die oberen Schwenkachsen sich durch die Aufnahmen hindurch erstrecken.

Teil der vorliegenden Erfindung ist auch eine Vorrichtung, die an der einen Aufnahme die Verbindung zur Führung mittels Verbindungselement, aufnahmeseitigem Arm, Kopplungselement und führungsseitigem Arm aufweist und an der anderen Aufnahme die Verbindung zur Führung mittels der Verzahnungsabschnitte aufweist.

Wie eingangs bereits erwähnt, so ist Teil der Erfindung auch ein System aus einer erfindungsgemäßen Vorrichtung sowie zwei Verlängerungsvorrichtungen, insbesondere und zwei Knochenankern. Insbesondere wobei die Vorrichtung derart ausgebildet ist, dass die unteren Schwenkachsen sich in dem Bereich der Verlängerungsvorrichtungen befinden, in dem die Verlängerungsvorrichtungen die Knochenanker kontaktieren, wenn sie an den Knochenankern angebracht sind. Im Sinne der Erfindung ist auch, wenn die unteren Schwenkachsen sich jeweils durch die Knochenanker erstrecken, wenn die Verlängerungsvorrichtungen mit den daran angebrachten Knochenankern in der Vorrichtung aufgenommen sind, wobei insbesondere vorgesehen sein kann, dass die Schwenkachse außerhalb der Verlängerungsvorrichtungen verlaufen. Vorzugsweise verlaufen die Schwenkachsen durch den Gabelkopf der, bspw. als Polyaxialschrauben ausgeführten, Knochenanker.

Weitere Merkmale, Einzelheiten und Vorteile der Erfindung ergeben sich aus den beigefügten Patentansprüchen und der zeichnerischen Darstellung und nachfolgenden Beschreibung einer bevorzugten Ausführungsform der erfindungsgemäßen Vorrichtung.

In der Zeichnung zeigt:
- Figur 1: eine Darstellung der erfindungsgemäßen Vorrichtung;
- Figur 2: eine perspektivische Ansicht der Vorrichtung aus Figur 1;
- Figur 3: eine weitere perspektivische Ansicht der Vorrichtung aus Figur 1;
- Figur 4: eine Ansicht der Vorrichtung aus Figur 1 in einer anderen Stellung;
- Figur 5: eine Ansicht der Vorrichtung aus Figur 1 in einer weiteren Stellung;
- Figur 6: eine Ansicht der Vorrichtung aus Figur 1 in einer weiteren Stellung;
- Figur 7: eine Ansicht der Vorrichtung aus Figur 1 in einer weiteren Stellung;
- Figur 8: eine Ansicht der Vorrichtung aus Figur 1 in einer weiteren Stellung;
- Figur 9: eine Ansicht der Vorrichtung aus Figur 1 in einer weiteren Stellung;
- Figur 10: eine Ansicht der Vorrichtung aus Figur 1 in einer weiteren Stellung;
- Figur 11: eine Ansicht der Vorrichtung aus Figur 1 in einer weiteren Stellung;
- Figur 12: eine Ansicht eines Teils der Vorrichtung aus Figur 1;
- Figur 13: eine Ansicht des Teils aus Figur 12 in teilweise explodierter Darstellung;
- Figur 14: eine Ansicht eines Teils der Vorrichtung aus Figur 1;
- Figur 15: eine weitere Ansicht des Teils der Vorrichtung aus Figur 14;
- Figur 16: eine weitere Ansicht des Teils der Vorrichtung aus Figur 14;
- Figur 17: eine Ansicht des Teils der Vorrichtung aus Figur 16 in explodierter Darstellung;
- Figur 18: eine Darstellung einer weiteren erfindungsgemäßen Vorrichtung;
- Figur 19: eine Ansicht der Vorrichtung aus Figur 18 in einer anderen Stellung;
- Figur 20: eine Ansicht der Vorrichtung aus Figur 18;
- Figur 21: eine Ansicht der Vorrichtung aus Figur 18 in einer weiteren Stellung;
- Figur 22: eine Ansicht eines Teils der Vorrichtung aus Figur 18;
- Figur 23: eine Ansicht eines Teils der Vorrichtung aus Figur 18;
- Figur 24: eine Ansicht eines Teils der Vorrichtung aus Figur 18;
- Figur 25: eine Ansicht eines Teils der Vorrichtung aus Figur 18; und
- Figur 26: eine Ansicht eines Teils der Vorrichtung aus Figur 12.

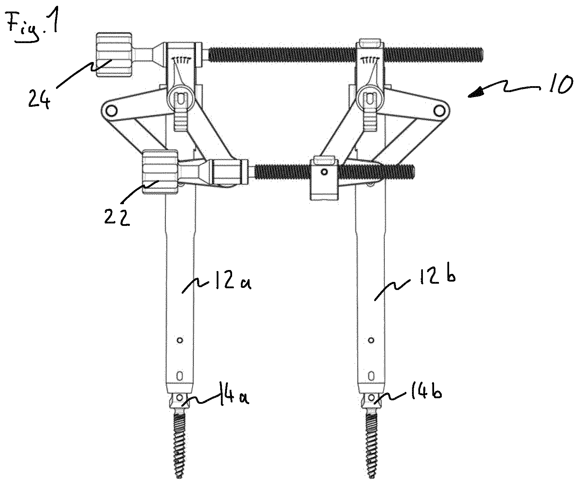

Figur 1 zeigt eine erfindungsgemäße Vorrichtung 10, die mit einer ersten Verlängerungsvorrichtung 12a und einer zweiten Verlängerungsvorrichtung 12b verbunden ist. Die erste Verlängerungsvorrichtung 12a ist mit einer ersten Knochenschraube 14a verbunden und die zweite Verlängerungsvorrichtung 12b ist mit einer zweiten Knochenschraube 14b verbunden.

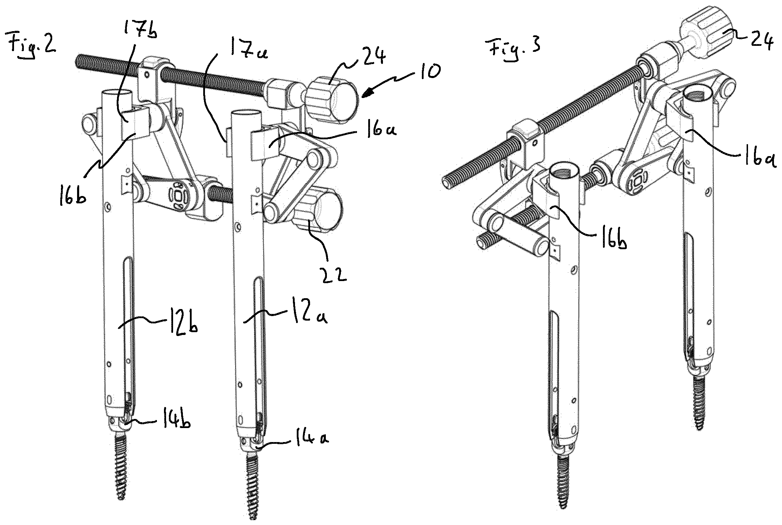

In den Figuren 2 und 3 ist die Vorrichtung 10 aus Figur 1 in verschiedenen perspektivischen Ansichten dargestellt. Die Vorrichtung 10 weist eine erste Aufnahme 16a, in der die erste Verlängerungsvorrichtung 12a aufgenommen ist, auf. Beabstandet zu der ersten Aufnahme 16a ist eine zweite Aufnahme 16b angeordnet, in der die zweite Verlängerungsvorrichtung 12b aufgenommen ist. Die Aufnahmen 16 sind bspw. in Figur 2 gut erkennbar.

Die erste Aufnahme 16a bildet einen ersten Krafteinleitungsabschnitt 17a in die erste Verlängerungsvorrichtung 12a, die zweite Aufnahme 16b wiederum bildet einen zweiten Krafteinleitungsabschnitt 17b in die zweite Verlängerungsvorrichtung 12b.

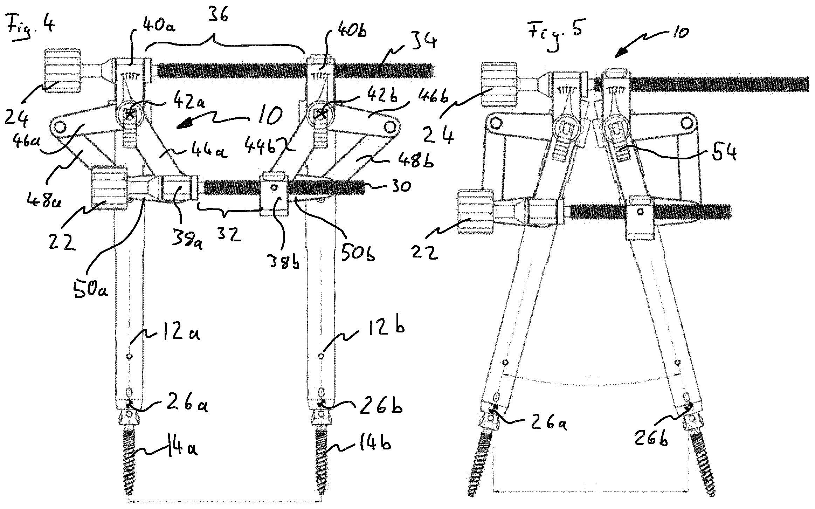

Die Vorrichtung 10 weist eine erste Betätigungseinrichtung 22 und eine zweite Betätigungseinrichtung 24 auf. Über die zweite Betätigungseinrichtung 24 sind die erste Verlängerungsvorrichtung 12a und die zweite Verlängerungsvorrichtung 12b jeweils um eine erste untere Schwenkachse 26a bzw. eine zweite untere Schwenkachse 26b verschwenkbar. Dabei bleibt bei alleiniger Betätigung der zweiten Betätigungseinrichtung 24 der Abstand zwischen den unteren Schwenkachsen 26 konstant. Die Lage der unteren Schwenkachsen 26a, 26b ist in Figur 4 gut erkennbar. Im vorliegenden Beispiel ist die Vorrichtung 10 derart ausgebildet, dass die untere Schwenkachsen 26 in dem Bereich der Verlängerungsvorrichtungen 12 angeordnet ist, in dem die Knochenschrauben 14 mit den Verlängerungsvorrichtungen 12 gekoppelt sind. Denkbar ist jedoch beispielsweise auch, dass die unteren Schwenkachse 26 quasi außerhalb der Verlängerungsvorrichtungen 12 liegen. Beispielsweise ist es möglich, dass die unteren Schwenkachse 26 im Bereich des Übergangs zwischen dem Gabelkopf und dem gewindetragenden Teil der Knochenschrauben 14 angeordnet sind. Das Verschwenken um die unteren Schwenkachsen 26a, 26b ist in Figuren 4 und 5 illustriert.

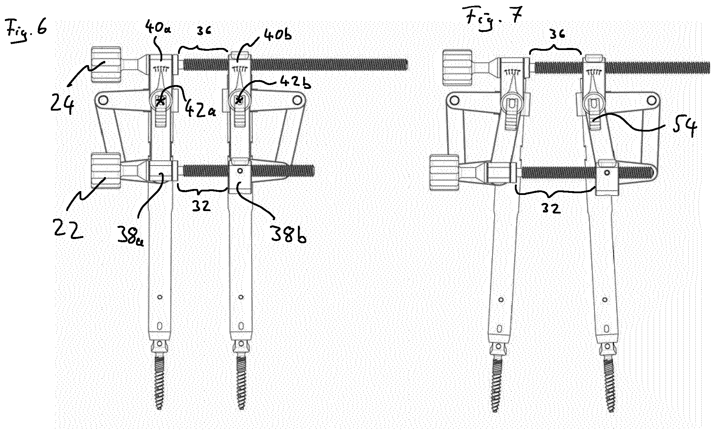

Über die erste Betätigungseinrichtung 22 sind die erste Verlängerungsvorrichtung 12a und die zweite Verlängerungsvorrichtung 12b bei Betätigung der Betätigungseinrichtung 22 derart zueinander bewegbar, dass sich ein Abstand zwischen der ersten unteren Schwenkachse 26a und der zweiten unteren Schwenkachse 26b verändert. Vorliegend sind über die erste Betätigungseinrichtung 22 die erste Verlängerungsvorrichtung 12a und die zweite Verlängerungsvorrichtung 12b jeweils um eine erste obere Schwenkachse 42a bzw. eine zweite obere Schwenkachse 42b verschwenkbar. Das Verschwenken um die oberen Schwenkachsen 42a, 42b ist in den Figuren 6 und 7 illustriert. Vorliegend wird alos mittels des Verschwenkens der Verlängerungsvorrichtungen 12a und 12b um die oberen Schwenkachsen 42a und 42b eine Abstandsänderung der unteren Schwenkachsen 26a und 26b bewirkt. Mit dieser Abstandsänderung kann der Abstand der Knochenanker 14a und 14b definiert eingestellt werden. Dabei bleibt bei alleiniger Betätigung der ersten Betätigungseinrichtung 22 der Abstand zwischen den oberen Schwenkachsen 42 konstant. Die in den Figuren 4 bis 7 dargestellten Schwenkbewegungen werden, wie bereits erläutert, durch Betätigung der ersten Betätigungseinrichtung 22 bzw. der zweiten Betätigungseinrichtung 24 vorgenommen. Der über die Betätigungseinrichtungen 22, 24 bedienbare Mechanismus zur Durchführung der Schwenkbewegungen wird nachfolgend erläutert.

Die erste Betätigungseinrichtung 22 ist mit einem ersten Stellelement 30 verbunden, das einen längenveränderbaren Abschnitt 32 aufweist. Das erste Stellelement 30 ist vorliegend als Gewindestange ausgebildet.

Die zweite Betätigungseinrichtung 24 ist mit einem zweiten Stellelement 34 verbunden, das einen längenveränderbaren Abschnitt 36 aufweist. Das zweite Stellelement 34 ist in der vorliegenden Ausführungsform als Gewindestange ausgebildet.

Das erste Stellelement 30 ist in einer der ersten Verlängerungsvorrichtung 12a zugeordneten Führung 38a sowie in einer der zweiten Verlängerungsvorrichtung 12b zugeordneten Führung 38b, die ein Innengewinde trägt, gelagert.

Der längenveränderbare Abschnitt 32 des ersten Stellelements 30 erstreckt sich von der der ersten Verlängerungsvorrichtung 12a zugeordneten Führung 38a zu der der zweiten Verlängerungsvorrichtung 12b zugeordneten Führung 38b.

Das zweite Stellelement 34 ist in einer der ersten Verlängerungsvorrichtung 12a zugeordneten weiteren Führung 40a sowie in einer der zweiten Verlängerungsvorrichtung 12b zugeordneten weiteren Führung 40b gelagert.

Der längenveränderbare Abschnitt 36 des zweiten Stellelements 34 erstreckt sich von der der ersten Verlängerungsvorrichtung 12a zugeordneten weiteren Führung 40a zu der der zweiten Verlängerungsvorrichtung 12b zugeordneten weiteren Führung 40b.

Von der ersten Aufnahme 16a erstreckt sich ein erstes Verbindungselement 44a zu der der ersten Verlängerungsvorrichtung 12a zugeordneten Führung 38a. Entsprechend erstreckt sich von der zweiten Aufnahme 16b ein zweites Verbindungselement 44b zu der der zweiten Verlängerungsvorrichtung 12b zugeordneten Führung 38b. Die jeweiligen Verbindungselemente 44a und 44b sind in sich starr ausgebildet.

Die Verbindungselemente 44a und 44b sind jeweils über einen ersten aufnahmeseitigen Arm 46a bzw. einen zweiten aufnahmeseitigen Arm 46b mit einem ersten bzw. zweiten Kopplungselement 48a bzw. 48b verbunden, wobei sich das jeweilige Kopplungselement 48a bzw. 48b von der Verbindungsstelle mit dem jeweiligen aufnahmeseitigen Arm 46a bzw. 46b zu einer Verbindungsstelle mit einem jeweiligen ersten bzw. zweiten führungsseitigen Arm 50a bzw. 50b erstreckt.

Die jeweiligen Verbindungselemente 44a und 44b sind um die jeweiligen Führungen 38a bzw. 38b sowie die Aufnahmen 16a bzw. 16b schwenkbar. Die aufnahmeseitigen Arme 46, Kopplungselemente 48 und führungsseitigen Arme 50 sind jeweils schwenkbar miteinander sowie schwenkbar mit dem jeweiligen Verbindungselement 44 verbunden.

Die aufnahmeseitigen Arme 46 sind mit den jeweiligen Aufnahmen 16 drehfest verbunden. Die führungsseitigen Arme 50 wiederum sind drehfest mit den Führungen 38 verbunden.

Durch die Anordnung der Verbindungselemente 44, der aufnahmeseitigen Arme 46, der Kopplungselemente 48 sowie der führungsseitigen Arme 50 sind die Führungen 38 sowie die weiteren Führungen 40 mit den Aufnahmen 16 gekoppelt. Durch diese Kopplung kann durch eine Veränderung des Abstands der Führungen 38 bzw. der weiteren Führungen 40 zueinander eine jeweilige Schwenkbewegung Verlängerungsvorrichtungen 12 um die unteren und oberen Schwenkachsen 26 und 42 bewirkt werden. Die Schwenkbewegung wird dabei durch eine entsprechende Krafteinleitung über die Aufnahmen 16 in die Verlängerungsvorrichtungen 12 bewirkt.

Vorliegend sind die Betätigungseinrichtungen 22 und 24 derart ausgeführt, dass durch eine Drehung der Betätigungseinrichtung 22 und 24 die Stellelemente 30 und 34 rotierbar sind. Die Stellelemente 30 und 34 sind in der der ersten Verlängerungsvorrichtung 12a zugeordneten Führung 38a sowie der weiteren Führung 40a rotierbar jedoch translatorisch gehalten gelagert. In der der zweiten Verlängerungsvorrichtung 12b zugeordneten Führung 38b sowie der weiteren Führung 40b sind das erste Stellelement 30 und das zweite Stellelement 34 in einem in der Führung 38b bzw. weiteren Führung 40b integrierten Gewinde rotierbar und translatorisch gegenüber der Führung 38b bzw. weiteren Führung 40b verschieblich (durch Verschrauben in dem Gewinde) gelagert.

Bei Drehung der Stellelemente 30 bzw. 34 über die Betätigungseinrichtung 22 bzw. 24 schrauben sich die Stellelemente 30 bzw. 34 in die Gewinde der Führung 38b bzw. weiteren Führung 40b. Hierdurch wird der Abstand zwischen den Führungen 38a und 38b bzw. weiteren Führungen 40a und 40b, die der ersten und zweiten Verlängerungsvorrichtung 12a und 12b zugeordnet sind jeweils verändert. Mit anderen Worten der längenveränderbare Abschnitt 32 des ersten Stellelements 30 wird hierdurch verändert, sodass der Abstand zwischen der der ersten Verlängerungsvorrichtung 12a zugeordneten Führung 38a sowie der der zweiten Verlängerungsvorrichtung 12b zugeordneten Führung 38b verändert wird.

Entsprechend wird beim Einschrauben des Stellelements 34 in das Gewinde der weiteren Führung 40b die Länge des längenveränderbaren Abschnitts 36 des zweiten Stellelements 34 und damit der Abstand zwischen der der ersten Verlängerungsvorrichtung 12a zugeordneten weiteren Führung 40a und der der zweiten Verlängerungsvorrichtung 12b zugeordneten weiteren Führung 40b verändert.

Es kann bei den erfindungsgemäßen Vorrichtungen vorgesehen sein, dass eine Stabilisierungseinrichtung vorgesehen ist, die derart ausgebildet und angeordnet ist, dass die Stellelemente 30 und 34 durch die Stabilisierungseinrichtung stets in paralleler Ausrichtung gehalten werden. Bspw. kann die Stabilisierungseinrichtung zwei Stabilisierungsführungen umfassen, durch die sich jeweils ein Stellelement 30, 34 erstreckt, wobei diese Stabilisierungsführungen derart miteinander verbunden sind, dass die Stellelemente 30, 34 zwangsweise stets parallel zueinander ausgerichtet sind.

Die oberen Schwenkachsen 42 erstrecken sich im vorliegenden Beispiel durch die jeweiligen Aufnahmen 16 hindurch. Beim reinen Verschwenken um die oberen Schwenkachsen 42 (Figur 6 und 7) drehen sich also lediglich die Aufnahmen 16. Dies wird durch die oben beschriebene Längenveränderung des längenveränderbaren Abschnitts 32 des ersten Stellelements 30 bei gleichbleibender Länge des längenveränderbaren Abschnitts 36 des zweiten Stellelements 34 erreicht. Die Veränderung des Abstands zwischen der der ersten Verlängerungsvorrichtung 12a zugeordneten Führung 38a sowie der der zweiten Verlängerungsvorrichtung 12b zugeordneten Führung 38b bewirkt die Verdrehung der Aufnahmen 16, wobei deren Abstand über die drehbare Verbindung der jeweiligen weiteren Führung 40 mit der entsprechenden Aufnahme 16 konstant gehalten wird. Die Abstandsänderung zwischen den Führungen 38 wird über die Kopplung mit den Aufnahmen mittels den Verbindungselementen 44, den aufnahmeseitigen Armen 46, den Kopplungselementen 48 sowie den führungsseitigen Armen 50 in die Drehung der Aufnahmen gewandelt.

Beim reinen Verschwenken um die unteren Schwenkachsen 26 (Figur 4 und 5) drehen sich die Aufnahmen 16 und bewegen sich voneinander weg bzw. aufeinander zu. Dies wird vorliegend durch die oben beschriebene Längenveränderung des längenveränderbaren Abschnitts 36 des zweiten Stellelements 34 bei gleichbleibender Länge des längenveränderbaren Abschnitts 32 des ersten Stellelements 30 erreicht. Die Veränderung des Abstands zwischen den weiteren Führungen 40 überträgt sich durch deren drehbare Kopplung mit den Aufnahmen 16 auf diese. Der während dieser Abstandsänderung konstant gehaltene Abstand zwischen den Führungen 38, die wiederum über das Verbindungselement 44, den aufnahmeseitigen Arm 46, das Kopplungselement 48 sowie den führungsseitigen Arm 50, mit der Aufnahme 16 gekoppelt sind, führt zu der Drehung der Aufnahmen 16, die ihrer Abstandänderung überlagert ist. Mit dieser Bewegung wird der Abstand der unteren Schwenkachsen 26a und 26b zueinander konstant gehalten.

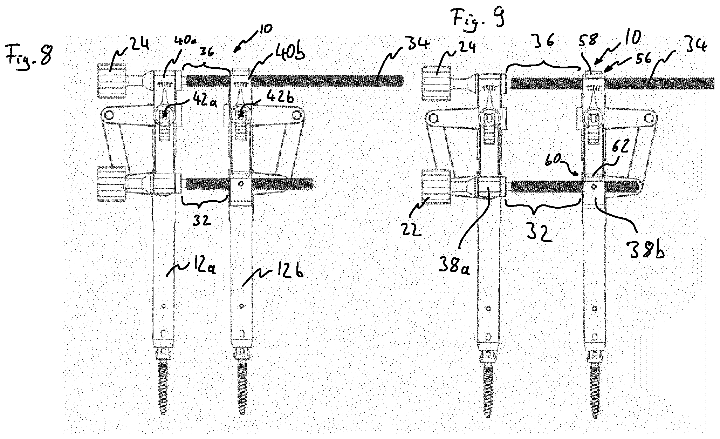

Werden beide Betätigungseinrichtungen 22 und 24 gleichzeitig betätigt, so können die Verlängerungsvorrichtungen 12a und 12b ohne ein Verschwenken zu bewirken aufeinander zu bzw. voneinander weg bewegt werden. Eine derartige Parallelverschiebung der Verlängerungsvorrichtungen 12 ist in den Figuren 8 und 9 illustriert. Die Vorrichtung 10 des vorliegenden Beispiels ist derart ausgelegt, dass sich, wenn die Längenänderung der beiden längenveränderbaren Abschnitte 32 bzw. 36 der Stellelemente 30, 34 sich unterscheidet, ein Verschwenken die Verlängerungsvorrichtungen 12a bzw. 12b um die entsprechenden Schwenkachsen eintritt. Dieses Verschwenken kann der Parallelverschiebung der Verlängerungsvorrichtungen 12 überlagert sein.

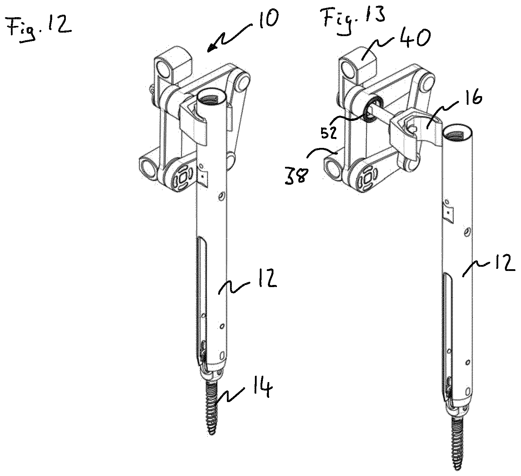

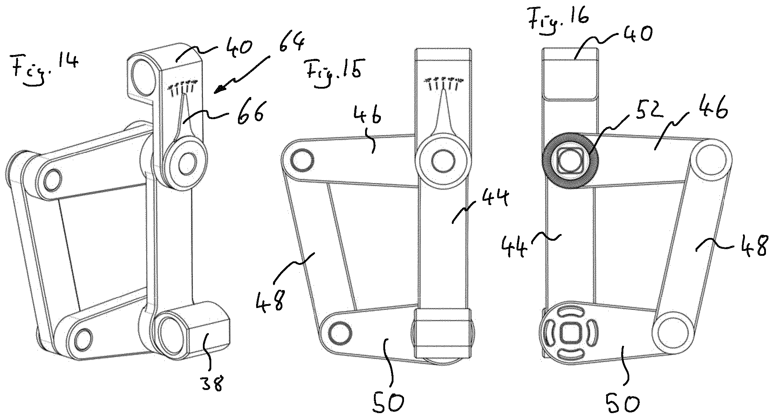

In den Figuren 12 und 13 ist ein Teil der Vorrichtung 10 mit einer Verlängerungsvorrichtung 12 einzeln dargestellt. In Figur 12 ist die drehfeste Verbindung zwischen dem aufnahmeseitigen Arm 46 und der Aufnahme 16 gut erkennbar. Die drehfeste Verbindung ist vorliegend über eine Verzahnung 52 auf Seiten des aufnahmeseitigen Arms 46 sowie eine entsprechende Verzahnung auf Seiten der Aufnahme realisiert (Siehe hierzu auch Figur 16).

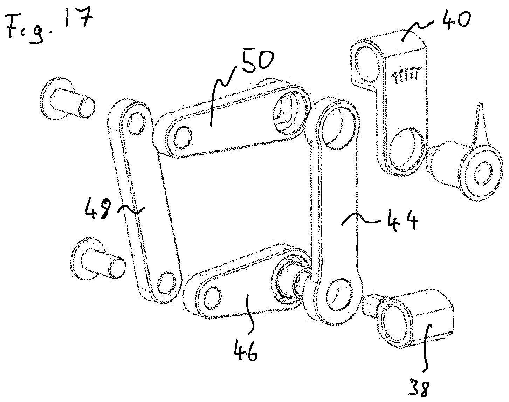

In den Figuren 14 bis 16 ist die Anordnung der Verbindungselemente 44, der aufnahmeseitigen Arme 46, der Kopplungselemente 48 und der führungsseitigen Arme 50 nochmal im Detail gezeigt und in Figur 17 in einer Explosionsdarstellung gezeigt.

Die Vorrichtung 10 weist einen Lösemechanismus 54 auf. Über den Lösemechanismus 54 kann der Eingriff der Verzahnung 52 auf Seiten des aufnahmeseitigen Arms 46 mit der Aufnahme 16 gelöst werden, sodass die drehfeste Verbindung zwischen dem aufnahmeseitigen Arm 46 in der Aufnahme 16 temporär lösbar ist, sodass die Aufnahme 16 gegenüber dem aufnahmeseitigen Arm 46. Hierdurch kann die Position der Aufnahme 16 gegenüber dem aufnahmeseitigen Arm 46 frei verändert werden, wodurch die Schwenkstellung der Verlängerungsvorrichtung 12 angepasst werden kann ohne die Betätigungseinrichtungen 22 und 24 zu bedienen. Um eine Verschwenkung der Aufnahme 16 gegenüber der Neutralstellung durch Lösen der drehfeste Verbindung mit dem aufnahmeseitigen Arm 46 anzuzeigen, kann die Vorrichtung 10, wie im vorliegenden Beispiel, eine Anzeigeeinrichtung 64, beispielsweise umfassend einen drehfest mit dem aufnahmeseitigen Arm 46 verbundenen Zeiger 66, aufweisen.

Ein Lösemechanismus 54 kann auch im Bereich der Verbindung zwischen der Führung 38 und dem führungsseitigen Arm 50 angeordnet sein, um deren drehfeste Verbindung temporär zu lösen.

Die zweite Führung 38b und die zweite weitere Führung 40b weisen jeweils einen Freigangsmechanismus 56 bzw. 60 auf, die jeweils über einen Druckknopf 58 bzw. 62 betätigbar sind.

In der zweiten Führung 38b bzw. der zweiten weiteren Führung 40b sind das erste Stellelement 30 bzw. das zweite Stellelement 34 jeweils derart angeordnet, dass ihr Gewinde in ein führungsseitiges Gewinde eingreift. Beim Betätigen des Freigangmechanismus 56 bzw. 60 wird der Eingriff dieser Gewinde gelöst und das jeweilige Stellelement 30 bzw. 34 ist in der Führung 38b bzw. weiteren Führung 40b frei translatorisch bewegbar.

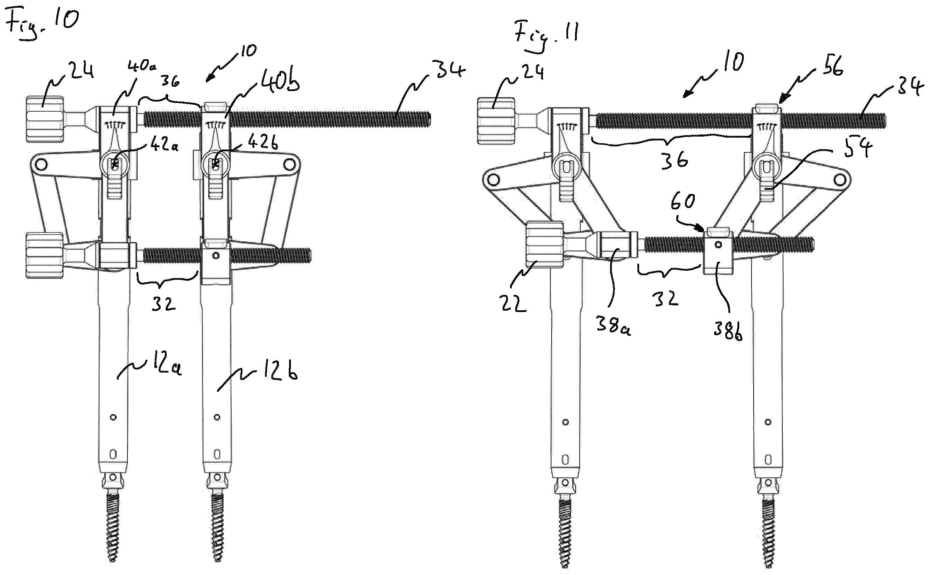

Durch lösen des Freigangsmechanismus 56 und des Lösemechanismus 54 kann die Vorrichtung 10 von der in Figur 10 gezeigten Stellung in die in Figur 11 gezeigte Stellung überführt werden, ohne dass die Betätigungseinrichtungen 22, 24 betätigt werden müssen.

In der klinischen Anwendung kann die Vorrichtung 10 bspw. wie folgt verwendet werden: Bei einem frakturierten Wirbelkörper kann zuerst der Abstand der Knochenschrauben 14 bzw. Pedikelschrauben, die in die benachbarten Wirbelkörper eingebracht sind, definiert eingestellt werden (Kompression/Distraktion). Im Anschluss kann die Schwenkstellung der Verlängerungsvorrichtungen geändert werden, ohne dass sich der Abstand der Knochenanker bzw. Pedikelschrauben ändert. Damit kann ein frakturierter Wirbelkörper, der meistens eine Keilfraktur erfahren hat, wieder aufgerichtet werden.

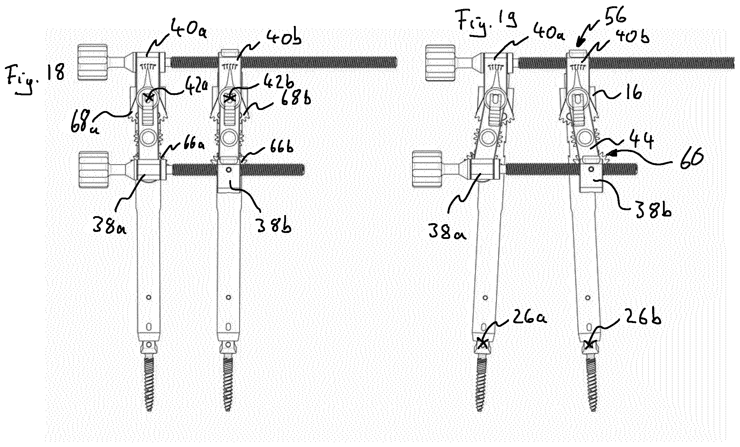

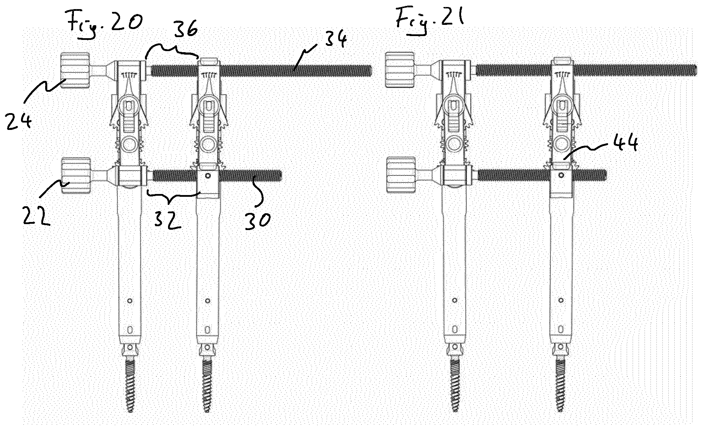

Figur 18-21 zeigen eine weitere erfindungsgemäße Ausführungsform der Vorrichtung 10.

Bei dieser Ausführungsform sind die Führungen 38 jeweils drehfest mit einem führungsseitigen Verzahnungsabschnitt 66 verbunden. Die Aufnahmen 16 sind jeweils drehfest mit einem aufnahmeseitigen Verzahnungsabschnitt 68 verbunden. Diese drehfeste Verbindung ist vorliegend über einen Lösemechanismus 54 lösbar ausgebildet. Die drehfeste Verbindung ist ähnlich wie bei der vorigen Ausführungsform über eine Verzahnung 52 ausgebildet die mit einer Verzahnung an der Aufnahme 16 in Eingriff gebracht werden kann. Die Verzahnung 52 ist dabei am aufnahmeseitigen Verzahnungsabschnitt 68 und an der Aufnahme angeordnet. Entsprechend ist dann im gelösten Zustand die Winkelstellung zwischen der Aufnahme 16 und dem aufnahmeseitigen Verzahnungsabschnitt 68 frei schwenkbar.

Auch die drehfeste Verbindung der Führung 38 mit dem führungsseitigen Verzahnungsabschnitt 66 kann über einen Lösemechanismus lösbar ausgebildet sein. Entsprechend ist dann im gelösten Zustand die Winkelstellung zwischen der Führung 38 und dem führungsseitigen Verzahnungsabschnitt 66 frei schwenkbar.

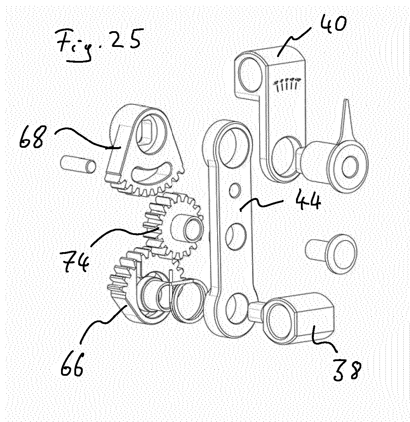

Der führungsseitige Verzahnungsabschnitt 66 und der aufnahmeseitige Verzahnungsabschnitt 68 sind derart miteinander bewegungsgekoppelt, dass der führungsseitige Verzahnungsabschnitt 66 und der aufnahmeseitige Verzahnungsabschnitt 68 gleichläufige Drehrichtungen 70 und 72 aufweisen. Die in Figur 25 durch die Pfeile illustrierte Drehrichtung ist dabei nur beispielhaft zu verstehen. Genauso kann die Drehung in die umgekehrte Richtung erfolgen, wobei sie dann ebenso für den führungsseitigen Verzahnungsabschnitt 66 und den aufnahmeseitigen Verzahnungsabschnitt 68 gleichgerichtet ist. Die Bewegungskopplung ist vorliegend über ein zwischen dem führungsseitigen Verzahnungsabschnitt 66 und dem aufnahmeseitigen Verzahnungsabschnitt 68 angeordnetes Zahnrad 74 realisiert. Das Zahnrad 74 weist eine gegenüber den Drehrichtungen 70 und 72 der Verzahnungsabschnitte 66 und 68 umgekehrte Drehrichtung 76 auf und steht mit den Verzahnungsabschnitten 66 und 68 in Eingriff.

Die Führungen 38 mit den jeweiligen führungsseitigen Verzahnungsabschnitten 66 sind jeweils über ein in sich starres, gegenüber der jeweiligen Aufnahme 16 und gegenüber der jeweiligen Führung 38 schwenkbares Verbindungselement 44, mit der entsprechenden Aufnahme 16 verbunden. Das Verbindungselement 44 ist ähnlich dem Verbindungselement 44 der vorigen Ausführungsform ausgebildet, länglich erstreckt und orthogonal zur jeweiligen oberen Schwenkachse 42 angeordnet.

An dem Verbindungselement 44 ist das Zahnrad 74 drehbar gelagert angeordnet.

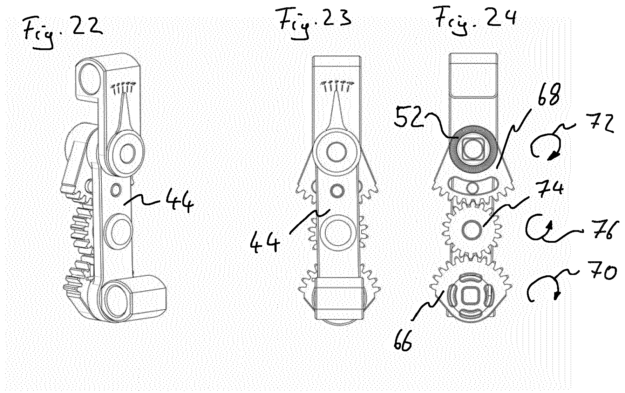

Ansonsten ist die Vorrichtung 10 der Figuren 18-25 ähnlich der vorigen Ausführungsform aufgebaut und weist ebenso eine erste Betätigungseinrichtung 22 und eine zweite Betätigungseinrichtung 24 sowie ein entsprechendes erstes Stellelement 30 und ein zweites Stellelement 34 mit entsprechenden längenveränderbaren Abschnitten 32 und 36 auf.

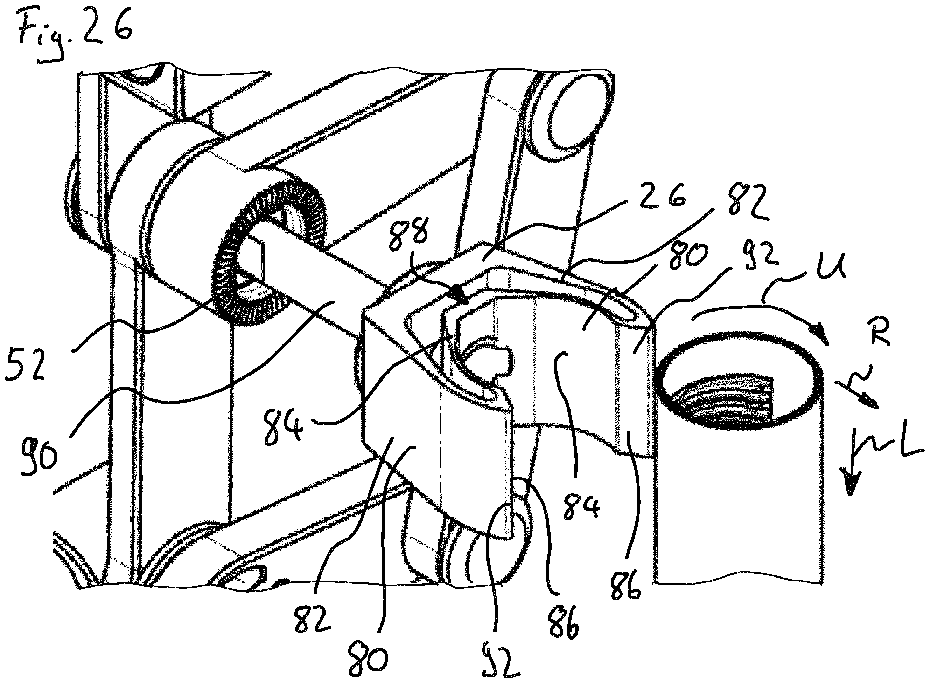

Die Aufnahmen 16 sind mit zwei entlang einer Umfangsrichtung U und Längsrichtung L der Verlängerungsvorrichtung 12 erstreckten, nachgiebig auslenkbaren Schenkeln 80 ausgebildet, welcher von außen an den Außenumfang der Verlängerungsvorrichtung 12 angelegt werden können (siehe Figur 26 die einen Teilbereich von Figur 13 vergrößert zeigt).

Die Schenkel weisen je eine Außenwand 82 und eine Innenwand 84 auf. Die Außenwand 82 und die Innenwand 84 sind in jedem der Schenkel 80 jeweils an wenigstens einer Stelle 86 (vorliegend dem umfänglichen Ende der Schenkel) so miteinander verbunden, dass beim Gegeneinanderziehen von Außenwand 82 und Innenwand 84 in radialer Richtung R der Verlängerungsvorrichtung 12 an einer Zugeinleitungsstelle 88 mittels eines Zugeinleitungsmechanismus 90 eine ausgehend von der Zugeinleitungsstelle 88 in der Umfangsrichtung U der Verlängerungsvorrichtung 12 wirkende Druckspannung in der Außenwand 82 und eine in entgegengesetzter Umfangsrichtung U wirkende Zugspannung in der Innenwand 84 erzeugt wird, wodurch der jeweilige Schenkel 80 gegen den Außenumfang der Verlängerungsvorrichtung 12 gezwungen wird und dadurch die Aufnahme 16 an der Verlängerungsvorrichtung 12 klemmend anordenbar ist bzw. die Verlängerungsvorrichtung 12 durch die Aufnahme 16 klemmend greifbar ist. Die Verbindung von Außenwand 82 und Innenwand 84 ist vorliegend im Bereich eines in Umfangsrichtung U freien Endes 92 des jeweiligen Schenkels realisiert. Die Außenwand 82 und die Innenwand 84 gehen vorliegend hierzu im Bereich des in Umfangsrichtung U freien Endes 92 des Schenkels 80 bzw. der Schenkel 80 einstückig ineinander über.

Vorliegend sind die Aufnahmen 16 derart ausgebildet, dass beidseitig von der Zugeinleitungsstelle 88 jeweils ein in der Umfangsrichtung U und in der Längsrichtung L der Verlängerungsvorrichtung erstreckter nachgiebig auslenkbarer Schenkel 80 vorgesehen ist.

## Patentansprüche

1. Vorrichtung (10) zur Korrektur einer Knochenfraktur unter Verwendung von Knochenankern (14), insbesondere Knochenschrauben (14), und an den Knochenankern (14) anbringbaren Verlängerungsvorrichtungen (12),
wobei die Vorrichtung (10) eine erste Aufnahme (16a) und eine zweite Aufnahme (16b) umfasst,
wobei die erste Aufnahme (16a) dazu ausgebildet und angeordnet ist, eine erste Verlängerungsvorrichtung (12a) für einen ersten Knochenanker (14a) aufzunehmen und einen ersten Krafteinleitungsabschnitt (17a) in die
erste Verlängerungsvorrichtung (12a) zu bilden, und wobei die zweite Aufnahme (16b) dazu ausgebildet und angeordnet ist, eine zweite Verlängerungsvorrichtung (12b) für einen zweiten Knochenanker (14b) aufzunehmen und einen zweiten Krafteinleitungsabschnitt (17b) in die zweite Verlängerungsvorrichtung (12b) zu bilden,
wobei die Vorrichtung eine erste Betätigungseinrichtung (22) und eine zweite Betätigungseinrichtung (24) umfasst,
wobei die Vorrichtung (10) derart ausgebildet ist, dass die erste Verlängerungsvorrichtung (12a) eine erste obere vorgesehene Schwenkachse (42a) und eine zu dieser beabstandete erste untere vorgesehene Schwenkachse (26a) aufweist und die zweite Verlängerungsvorrichtung (12b) eine zweite obere vorgesehene Schwenkachse (42b) und eine zu dieser beabstandete zweite untere vorgesehene Schwenkachse (26b) aufweist, wenn die Verlängerungsvorrichtungen (12) in den vorgesehenen Positionen in den Aufnahmen (16) aufgenommen sind,
wobei die erste Betätigungseinrichtung (22) ausgebildet ist, um bei Betätigung die erste Verlängerungsvorrichtung (12a) und die zweite Verlängerungsvorrichtung (12b) derart zueinander zu bewegen, dass sich ein Abstand zwischen der ersten unteren Schwenkachse (26a) und der zweiten unteren Schwenkachse (26b) verändert, insbesondere wobei die Änderung des Abstands durch ein Verschwenken der ersten Verlängerungsvorrichtung (12a) um die erste obere Schwenkachse (42a) und der zweiten Verlängerungsvorrichtung (12b) um die zweite obere Schwenkachse (42b) bewirkt wird, und
wobei die zweite Betätigungseinrichtung (24) ausgebildet ist, um bei deren Betätigung die erste Verlängerungsvorrichtung (12a) um die erste untere Schwenkachse (26a) und die zweite Verlängerungsvorrichtung (12b) um die zweite untere Schwenkachse (26b) zu verschwenken,
**dadurch gekennzeichnet, dass**
der durch die erste Aufnahme (16a) gebildete erste Krafteinleitungsabschnitt (17a) in Richtung der Längserstreckung der ersten Verlängerungsvorrichtung (12a) zu der ersten unteren Schwenkachse (26a), beabstandet ist und der durch die zweite Aufnahme (16b) gebildete zweite Krafteinleitungsabschnitt (17b) in Richtung der Längserstreckung (L) der zweiten Verlängerungsvorrichtung (12b) zu der zweiten unteren Schwenkachse (26b), beabstandet ist, wenn die Verlängerungsvorrichtungen (12) in den vorgesehenen Positionen in den Aufnahmen (16a, 16b) aufgenommen sind.

2. Vorrichtung nach Anspruch 1, **dadurch gekennzeichnet, dass** die erste Betätigungseinrichtung (22) ausgebildet ist, um die Abstandsänderung zwischen den unteren Schwenkachsen (26) bzw. die Schwenkbewegung um die oberen Schwenkachsen (42) durch eine Längenänderung eines längenveränderbaren Abschnitts (32) eines ersten Stellelements (30), insbesondere eines Abschnitts einer ersten Gewindestange, zu bewirken und/oder dass die zweite Betätigungseinrichtung (24) ausgebildet ist, um die Schwenkbewegung um die unteren Schwenkachsen (26) durch eine Längenänderungen eines längenveränderbaren Abschnitts (36) eines zweiten Stellelements (34), insbesondere eines Abschnitts einer zweiten Gewindestange, zu bewirken.

3. Vorrichtung nach Anspruch 2, **dadurch gekennzeichnet, dass** das erste Stellelement (30) in einer der ersten Verlängerungsvorrichtung (12a) zugeordneten Führung (38a) und in einer der zweiten Verlängerungsvorrichtung (12b) zugeordneten Führung (38b) gelagert ist und sich der längenveränderbare Abschnitt (32) des ersten Stellelements (30) von der der ersten Verlängerungsvorrichtung (12a) zugeordneten Führung (38a) bis zu der der zweiten Verlängerungsvorrichtung (12b) zugeordneten Führung (38b) erstreckt.

4. Vorrichtung nach Anspruch 2 oder 3, **dadurch gekennzeichnet, dass** das zweite Stellelement (34) in einer der ersten Verlängerungsvorrichtung (12a) zugeordneten weiteren Führung (40a) und in einer der zweiten Verlängerungsvorrichtung (12b) zugeordneten weiteren Führung (40b) gelagert ist und sich der längenveränderbare Abschnitt (36) des zweiten Stellelements (34) von der der ersten Verlängerungsvorrichtung (12a) zugeordneten weiteren Führung (40a) bis zu der der zweiten Verlängerungsvorrichtung (12b) zugeordneten weiteren Führung (40b) erstreckt.

5. Vorrichtung nach Anspruch 3 oder 4, **dadurch gekennzeichnet, dass** die der ersten Verlängerungsvorrichtung (12a) zugeordnete Führung (38a) um die erste Aufnahme (16a) schwenkbar ist und dass die der zweiten Verlängerungsvorrichtung (12b) zugeordnete Führung (38b) um die zweite Aufnahme (16b) schwenkbar ist.

6. Vorrichtung nach einem der Ansprüche 3 bis 5, **dadurch gekennzeichnet, dass** eine Führung (38) zu der entsprechenden Aufnahme (16) in einer zur entsprechenden oberen Schwenkachse (42) orthogonalen Ebene beabstandet angeordnet ist, insbesondere und über ein in sich starres, gegenüber der entsprechenden Aufnahme und gegenüber der entsprechenden Führung schwenkbares entsprechendes Verbindungselement (44), insbesondere das länglich erstreckt und orthogonal zur entsprechenden oberen Schwenkachse (42) ausgebildet ist, mit dieser verbunden ist, insbesondere dass beide Führungen (38) mit den jeweiligen Aufnahmen (16) auf diese Art beabstandet und verbunden sind.

7. Vorrichtung nach einem der Ansprüche 3 bis 6, **dadurch gekennzeichnet, dass** ein aufnahmeseitiger Arm (46) drehfest gegenüber einer der Aufnahmen (16) angeordnet ist und sich von der Aufnahme (16) zu einem von der Aufnahme (16) beabstandeten und schwenkbar mit dem Arm (46) verbundenen Kopplungselement (48) erstreckt, welches wiederum schwenkbar mit einem führungsseitigen Arm (50) verbunden ist, der sich wiederum von der schwenkbaren Verbindung mit dem Kopplungselement (48) zu der Führung (38) erstreckt und drehfest gegenüber dieser angeordnet ist, insbesondere dass beide Führungen (38) mit den jeweiligen Aufnahmen (16) auf diese Art verbunden sind, insbesondere wobei die drehfeste Verbindung zwischen der Aufnahme (16) und dem aufnahmeseitigen Arm (46) und/oder die drehfeste Verbindung zwischen der Führung (38) und dem führungsseitigen Arm (50) temporär lösbar ist, so dass die Winkelstellung zwischen der Aufnahme (16) und dem aufnahmeseitigen Arm (46) bzw. zwischen der Führung (38) und dem führungsseitigen Arm (50) frei schwenkbar ist.

8. Vorrichtung nach einem der Ansprüche 3 bis 7, **dadurch gekennzeichnet, dass** die der ersten Verlängerungsvorrichtung (12a) zugeordnete Führung (38a) oder die der zweiten Verlängerungsvorrichtung (12b) zugeordnete Führung (38b) drehfest mit einem führungsseitigen Verzahnungsabschnitt (66) verbunden ist und die entsprechende Aufnahme (16a, 16b) drehfest mit einem aufnahmeseitigen Verzahnungsabschnitt (68) verbunden ist, der über ein zwischen dem führungsseitigen Verzahnungsabschnitt (66) und dem aufnahmeseitigen Verzahnungsabschnitt (68) angeordnetes Zahnrad (74), insbesondere das mit dem führungsseitigen Verzahnungsabschnitt und dem aufnahmeseitigen Verzahnungsabschnitt in Eingriff steht, derart bewegungsgekoppelt ist, dass der führungsseitige Verzahnungsabschnitt (66) und der aufnahmeseitige Verzahnungsabschnitt (68) gleichläufige Drehrichtungen (70, 72) aufweisen, insbesondere dass beide Führungen (38a, 38b) mit den jeweiligen Aufnahmen (16a, 16b) auf diese Art verbunden sind.

9. Vorrichtung nach Anspruch 8, **dadurch gekennzeichnet, dass** die Führung (38) mit führungsseitigem Verzahnungsabschnitt (66) über ein in sich starres, gegenüber der entsprechenden Aufnahme und gegenüber der Führung schwenkbares Verbindungselement (44), insbesondere das länglich erstreckt und orthogonal zur entsprechenden oberen Schwenkachse (42) ausgebildet ist, mit der entsprechenden Aufnahme (16) verbunden ist, wobei an dem Verbindungselement (44) das Zahnrad (74) drehbar gelagert angeordnet ist, insbesondere dass beide Führungen (38) mit den jeweiligen Aufnahmen (16) auf diese Art beabstandet und verbunden sind.

10. Vorrichtung nach einem der beiden vorangegangenen Ansprüche, **dadurch gekennzeichnet, dass** die drehfeste Verbindung zwischen der Aufnahme (16) und dem aufnahmeseitigen Verzahnungsabschnitt (68) und/oder zwischen der Führung (38) und dem führungsseitigen Verzahnungsabschnitt (66) temporär lösbar ist, so dass die Winkelstellung zwischen der Aufnahme (16) und dem aufnahmeseitigen Verzahnungsabschnitt (68) bzw. zwischen der Führung (38) und dem führungsseitigen Verzahnungsabschnitt (66) frei schwenkbar ist.

11. Vorrichtung nach einem der vorangegangenen Ansprüche, **dadurch gekennzeichnet, dass** wenigstens eine Aufnahme (16) mit zwei entlang einer Umfangs- (U) und Längsrichtung (L) der Verlängerungsvorrichtung (12) erstreckten, nachgiebig auslenkbaren Schenkeln (80) ausgebildet ist, welcher von außen an den Außenumfang der Verlängerungsvorrichtung (12) angelegt werden kann, wobei die Schenkel (80) je eine Außenwand (82) und eine Innenwand (84) aufweisen, die an wenigstens einer Stelle (86) so miteinander verbunden sind, dass beim Gegeneinanderziehen von Außenwand (82) und Innenwand (84) in radialer Richtung (R) der Verlängerungsvorrichtung (12) an einer Zugeinleitungsstelle (88) mittels eines Zugeinleitungsmechanismus (90) eine ausgehend von der Zugeinleitungsstelle (88) in der Umfangsrichtung (U) der Verlängerungsvorrichtung (12) wirkende Druckspannung in der Außenwand (82) und eine in entgegengesetzter Umfangsrichtung (U) wirkende Zugspannung in der Innenwand (84) erzeugt wird, wodurch der jeweilige Schenkel (80) gegen den Außenumfang der Verlängerungsvorrichtung (12) gezwungen wird und dadurch die Aufnahme (16) an der Verlängerungsvorrichtung (12) klemmend anordenbar ist.

12. System umfassend eine Vorrichtung (10) nach einem der Ansprüche 1 bis 11 sowie zwei Verlängerungsvorrichtungen (12), die in den Aufnahmen (16) aufgenommen sind, insbesondere und zwei Knochenanker (14) mit je einem Gabelkopf, wobei die Vorrichtung (10) derart ausgebildet ist, dass die unteren Schwenkachsen (26) sich in einem Bereich der Verlängerungsvorrichtungen (12) befinden, in dem die Verlängerungsvorrichtungen (12) die Knochenanker (14) kontaktieren, wenn sie an den Knochenankern (14) angebracht sind, oder dass die unteren Schwenkachsen (26) sich jeweils durch die Knochenanker (14) erstrecken, wenn die Verlängerungsvorrichtungen (12) mit den daran angebrachten Knochenankern (14) in der Vorrichtung (10) aufgenommen sind, insbesondere wobei die unteren Schwenkachsen (26) durch den Gabelkopf verlaufen.

13. System, insbesondere nach Anspruch 12, umfassend eine Vorrichtung (10) nach einem der Ansprüche 1 bis 11 sowie zwei Verlängerungsvorrichtungen (12), die in den Aufnahmen (16) aufgenommen sind, wobei die Verlängerungsvorrichtungen (12) an ihrem unteren Ende, insbesondere im Bereich der unteren Schwenkachsen (26), zu der Vorrichtung (10) beabstandet sind und in diesem Bereich mit der Vorrichtung (10) nicht in Kontakt stehen.

## Claims

1. Device (10) for correcting bone fractures using bone anchors (14), in particular bone screws (14), and extension devices (12) attachable to the bone anchors (14),
wherein the device (10) comprises a first receptacle (16a) and a second receptacle (16b),
wherein the first receptacle (16a) is designed and arranged to receive a first extension device (12a) for a first bone anchor (14a) and to form a first force introduction section (17a) into the first extension device (12a), and
wherein the second receptacle (16b) is designed and arranged to receive a second extension device (12b) for a second bone anchor (14b) and to form a second force introduction section (17b) into the second extension device (12b),
wherein the device comprises a first actuating device (22) and a second actuating device (24),
wherein the device (10) is designed such that the first extension device (12a) has a first upper pivot axis (42a) provided and a first lower pivot axis (26a) provided at a distance from this and the second extension device (12b) has a second upper pivot axis provided (42b) and a second, lower pivot axis (26b) provided at a distance from this, when the extension devices (12) are received in the provided positions in the receptacles (16),
wherein the first actuating device (22) is designed to move, when actuated, the first extension device (12a) and the second extension device (12b) to one another in such a way that a distance between the first lower pivot axis (26a) and the second lower pivot axis (26b) changes, in particular wherein the change in the distance is caused by pivoting the first extension device (12a) about the first upper pivot axis (42a) and the second extension device (12b) about the second upper pivot axis (26b), and
wherein the second actuating device (24) is designed to pivot the first extension device (12a) about the first lower pivot axis (26a) and the second extension device (12b) about the second lower pivot axis (26b) when actuated,
**characterized in that**
the first force introduction section formed by the first receptacle (16a) is spaced apart in the direction of the longitudinal extent of the first extension device (12a, 12b) from the first lower pivot axes (26a) and that the second force introduction section (17b) formed through the second receptacle (16b) is spaced in the direction of the longitudinal extension (L) of the second extension device (12b) to the second lower pivot axis (26b), when the extension devices (12) are received in the intended positions in the receptacles (16) .

2. Device according to claim 1, **characterized in that** the first actuating device (22) is designed to adjust the change in distance between the lower pivot axes (26) or the pivot movement about the upper pivot axes (42) by changing the length of a length-adjustable section (32) of a first adjusting element (30), in particular a section of a first threaded rod, and/or that the second actuating device (24) is designed to perform the pivoting movement about the lower pivot axes (26) by changing the length of a length-adjustable section (36) of a second adjusting element (34), in particular a section of a second threaded rod.

3. Device according to claim 2, **characterized in that** the first adjusting element (30) is mounted in a guide (38a) assigned to the first extension device (12a) and in a guide (38b) assigned to the second extension device (12b) and the length-adjustable section (32) of the first adjusting element (30) extends from the guide (38a) assigned to the first extension device (12a) to the guide (38b) assigned to the second extension device (12b) .

4. Device according to claim 2 or 3, **characterized in that** the second adjusting element (34) is mounted in a further guide (40a) assigned to the first extension device (12a) and in a further guide (40b) assigned to the second extension device (12b) and the variable-length section (36) of the second adjusting element (34) extends from the further guide (40a) assigned to the first extension device (12a) to the further guide (40b) assigned to the second extension device (12b).

5. Device according to claim 3 or 4, **characterized in that** the guide (38a) assigned to the first extension device (12a) is pivotable about the first receptacle (16a) and that the guide (38b) assigned to the second extension device (12b) is pivotable about the second receptacle (16b) .

6. Device according to one of claims 3 to 5, **characterized in that** a guide (38) is arranged at a distance from the corresponding receptacle (16) in a plane orthogonal to the corresponding upper pivot axis (42), in particular and is connected to this via an inherently rigid connecting element (44) which is pivotable with respect to the corresponding receptacle and with respect to the corresponding guide, in particular which is elongated and is orthogonal to the corresponding upper pivot axis (42), in particular that both guides (38) are spaced in this way and connected with the respective receptacles (16) .

7. Device according to one of claims 3 to 6, **characterized in that** a receptacle-side arm (46) is arranged non-rotatably opposite one of the receptacles (16) and extends from the receptacle (16) to a coupling element (48) spaced from the receptacle (16) and connected pivotably with the arm (46), which coupling element in turn is pivotably connected to a guide-side arm (50), which in turn extends from the pivotable connection with the coupling element (48) to the guide (38) and is arranged non-rotatably relative to this, in particular that both guides (38) are connected to the respective receptacles (16) in this way, in particular the non-rotatable connection between the receptacle (16) and the receptacle-side arm (46) and/or the non-rotatable connection between the guide (38) and the guide-side arm (50) is temporarily detachable, so that the angular position between the receptacle (16) and the receptacle-side arm (46) or between the guide (38) and the guide-side arm (50) is freely pivotable.

8. Device according to one of the claims 3 to 7, **characterized in that** the guide (38a) assigned to the first extension device (12a) or the guide (38b) assigned to the second extension device (12b) is non-rotatably connected to a guide-side toothed section (66) and the corresponding receptacle (16) is non-rotatably connected to a receptacle-side toothed section (68) which, in particular via a gear (74) arranged on the guide-side toothed section (66) and the receptacle-side toothed section (68), in particular which is in engagement with the guide-side toothed section and the receptacle-side toothed section, is coupled in terms of movement such that the guide-side toothed section (66) and the receptacle-side toothed section (68) have co-rotating directions of rotation (70, 72), in particular that both guides (38) are connected to the respective receptacles (16) in this way.

9. Device according to claim 8, **characterized in that** the guide (38) with the guide-side toothed section (66) extends via an inherently rigid connecting element (44) that can be pivoted relative to the corresponding receptacle and relative to the guide, in particular is formed elongated and orthogonal to the corresponding upper pivot axis (42), is connected to the corresponding receptacle (16), the gear (74) being rotatably mounted on the connecting element (44), in particular that both guides (38) are spaced and connected with the respective receptacles (16) in this way.

10. Device according to one of the two preceding claims, **characterized in that** the non-rotatable connection between the receptacle (16) and the receptacle-side toothed section (68) and/or between the guide (38) and the guide-side toothed section (66) is temporarily detachable, so that the angular position between the receptacle (16) and the receptacle-side toothed section (68) or between the guide (38) and the guide-side toothed section (66) is freely pivotable.

11. Device according to one of the preceding claims, **characterized in that** at least one receptacle (16) is designed with a resiliently deflectable leg (80) extending along a circumferential (U) and longitudinal direction (L) of the extension device (12), which leg (80) can be placed from the outside on the outer circumference of the extension device (12), the at least one leg (80) having an outer wall (82) and an inner wall (84), which are connected to one another at at least one point (86) in such a way that when pulling outer wall (82) and inner wall (84) against one another in the radial direction (R) of the extension device (12), at a tension introduction point (88) by means of a tension introduction mechanism (90), a compressive stress in the outer wall (82) acting in the circumferential direction (U) of the extension device (12) starting from the tension introduction point (88), and a tensile stress in the inner wall (84) acting in the opposite circumferential direction (U) is generated, whereby the leg (80) is forced against the outer circumference of the extension device (12) and the receptacle (16) can thereby be arranged in a clamping manner on the extension device (12).

12. System comprising a device (10) according to one of claims 1 to 11 and two extension devices (12) which are received in the receptacles (16), in particular and two bone anchors (14) each with a fork head, the device (10) being designed such that the lower pivot axes (26) are located in a region of the extension devices (12) in which the extension devices (12) contact the bone anchors (14) when they are attached to the bone anchors (14), or that the lower pivot axes (26) each extend through the bone anchors (14) when the extension devices (12) with the bone anchors (14) attached are received in the device (10), in particular wherein the lower pivot axes (26) run through the fork head, in particular outside of the extension devices (12).

13. System, in particular according to claim 12, comprising a device (10) according to one of claims 1 to 11 and two extension devices (12), which are received in the receptacles (16), wherein the extension devices (12) at their lower end, in particular in the area of the lower pivot axes (26), are spaced from the device (10) and are not in contact with the device (10) in this area.

## Revendications

1. Dispositif (10) de correction d'une fracture osseuse avec utilisation d'ancrages osseux (14), en particulier de vis à os (14), et de dispositifs de prolongation (12) pouvant être mis en place sur les ancrages osseux (14),
le dispositif (10) comprenant un premier logement (16a) et un deuxième logement (16b),
le premier logement (16a) étant constitué et disposé pour loger un premier dispositif de prolongation (12a) pour un premier ancrage osseux (14a) et pour former un premier segment d'application de force (17a) dans le premier dispositif de prolongation (12a), et
le deuxième logement (16b) étant constitué et disposé pour loger un deuxième dispositif de prolongation (12b) pour un deuxième ancrage osseux (14b) et pour former un deuxième segment d'application de force (17b) dans le deuxième dispositif de prolongation (12b),
le dispositif comprenant un premier moyen d'actionnement (22) et un deuxième moyen d'actionnement (24),
le dispositif (10) étant constitué de telle façon que le premier dispositif de prolongation (12a) présente un premier axe de pivotement (42a) prévu supérieur et un premier axe de pivotement (26a) prévu inférieur espacé de celui-ci, et le deuxième dispositif de prolongation (12b) présentant un deuxième axe de pivotement (42b) prévu supérieur et un deuxième axe de pivotement (26b) prévu inférieur espacé de celui-ci quand les dispositifs de prolongation (12) sont logés dans les positions prévues dans les logements (16),
le premier moyen d'actionnement (22) étant constitué pour, lors de l'actionnement, déplacer l'un par rapport à l'autre le premier dispositif de prolongation (12a) et le deuxième dispositif de prolongation (12b) de telle sorte qu'une distance entre le premier axe de pivotement (26a) inférieur et le deuxième axe de pivotement (26b) inférieur est modifié, la modification de la distance étant en particulier provoquée par un pivotement du premier dispositif de prolongation (12a) autour du premier axe de pivotement (42a) supérieur et par un pivotement du deuxième dispositif de prolongation (12b) autour du deuxième axe de pivotement (42b) supérieur, et le deuxième moyen d'actionnement (24) étant constitué pour, lors de son actionnement, faire pivoter le premier dispositif de prolongation (12a) autour du premier axe de pivotement (26a) inférieur et le deuxième dispositif de prolongation (12b) autour du deuxième axe de pivotement (26b) inférieur,
**caractérisé en ce que**
le premier segment d'application de force (17a) formé par le premier logement (16a) est, en direction de l'extension longitudinale du premier dispositif de prolongation (12a), espacé du premier axe de pivotement (26a) inférieur, et le deuxième segment d'application de force (17b) formé par le deuxième logement (16b) est, en direction de l'extension longitudinale (L) du deuxième dispositif de prolongation (12b), espacé du deuxième axe de pivotement (26b) inférieur quand les dispositifs de prolongation (12) sont logés dans les positions prévues dans les logements (16a, 16b).

2. Dispositif selon la revendication 1, **caractérisé en ce que** le premier moyen d'actionnement (22) est constitué pour provoquer la modification de distance entre les axes de pivotement (26) inférieurs ou respectivement le mouvement le pivotement autour des axes de pivotement (42) supérieurs par une modification de longueur d'un segment (32) modifiable en longueur d'un premier élément de réglage (30), en particulier d'un segment d'une première tige filetée et/ou **en ce que** le deuxième moyen d'actionnement (24) est constitué pour provoquer le mouvement de pivotement autour des axes de pivotement (26) inférieurs par une modification de longueur d'un segment (36) modifiable en longueur d'un deuxième élément de réglage (34), en particulier d'un segment d'une deuxième tige filetée.

3. Dispositif selon la revendication 2, **caractérisé en ce que** le premier élément de réglage (30) est supporté dans un guide (38a) affecté au premier dispositif de prolongation (12a) et dans un guide (38b) affecté au deuxième dispositif de prolongation (12b), et **en ce que** le segment (32) modifiable en longueur du premier élément de réglage (30) s'étend à partir du guide (38a) affecté au premier dispositif de prolongation (12a) jusqu'au guide (38b) affecté au deuxième dispositif de prolongation (12b).

4. Dispositif selon la revendication 2 ou 3, **caractérisé en ce que** le deuxième élément de réglage (34) est supporté dans un autre guide (40a) affecté au premier dispositif de prolongation (12a) et dans un autre guide (40b) affecté au deuxième dispositif de prolongation (12b), et **en ce que** le segment (36) modifiable en longueur du deuxième élément de réglage (34) s'étend à partir de l'autre guide (40a) affecté au premier dispositif de prolongation (12a) jusqu'à l'autre guide (40b) affecté au deuxième dispositif de prolongation (12b).

5. Dispositif selon la revendication 3 ou 4, **caractérisé en ce que** le guide (38a) affecté au premier dispositif de prolongation (12a) peut pivoter autour du premier logement (16a) et **en ce que** le guide (38b) affecté au deuxième dispositif de prolongation (12b) peut pivoter autour du deuxième logement (16b).

6. Dispositif selon l'une des revendications 3 à 5, **caractérisé en ce qu'**un guide (38) est disposé à distance du logement (16) correspondant dans un plan orthogonal à l'axe de pivotement (42) supérieur correspondant, et en particulier est, par le biais d'un élément de raccordement (44) correspondant rigide en soi qui peut pivoter par rapport au logement correspondant, qui s'étend en particulier en longueur et qui est constitué de façon orthogonale à l'axe de pivotement (42) supérieur correspondant, raccordé à cet axe de pivotement, en particulier **en ce que** les deux guides (38) sont à distance et raccordés de cette façon aux logements (16) respectifs.

7. Dispositif selon l'une des revendications 3 à 6, **caractérisé en ce qu'**un bras (46) côté logement est disposé bloqué en rotation par rapport à l'un des logements (16) et s'étend à partir du logement (16) jusqu'à un élément d'accouplement (48) espacé du logement (16) et raccordé de façon pivotante au bras (46), lequel élément d'accouplement est à son tour raccordé de façon pivotante à un bras (50) côté guide qui s'étend à son tour à partir du raccordement pivotant à l'élément d'accouplement (48) jusqu'au guide (38) et est disposé bloqué en rotation par rapport à celui-ci, en particulier **en ce que** les deux guides (38) sont raccordés de cette façon aux logements (16) respectifs, le raccordement bloqué en rotation entre le logement (16) et le bras (46) côté logement et/ou le raccordement bloqué en rotation entre le guide (38) et le bras (50) côté guide pouvant en particulier être débloqué temporairement de sorte que la position angulaire entre le logement (16) et le bras (46) côté logement ou respectivement entre le guide (38) et le bras (50) côté guide peut pivoter librement.

8. Dispositif selon l'une des revendications 3 à 7, **caractérisé en ce que** le guide (38a) affecté au premier dispositif de prolongation (12a) ou le guide (38b) affecté au deuxième dispositif de prolongation (12b) est raccordé bloqué en rotation à un segment de denture (66) côté guide, et le logement (16a, 16b) correspondant est raccordé bloqué en rotation à un segment de denture (68) côté logement qui, par le biais d'une roue dentée (74), disposée entre le segment de denture (66) côté guide et le segment de denture (68) côté logement et qui en particulier est en prise avec le segment de denture côté guide et avec le segment de denture côté logement, est couplé en déplacement de telle sorte que le segment de denture (66) côté guide et le segment de denture (68) côté logement présentent des sens de rotation (70, 72) identiques, en particulier **en ce que** les deux guides (38a, 38b) sont raccordés de cette façon aux logements (16a, 16b) respectifs.

9. Dispositif selon la revendication 8, **caractérisé en ce que** le guide (38) avec segment de denture (66) côté guide est raccordé au logement (16) correspondant par le biais d'un élément de raccordement (44) rigide en soi pouvant pivoter par rapport au logement correspondant et par rapport au guide, élément de raccordement qui en particulier s'étend en longueur et est constitué de façon orthogonale par rapport à l'axe de pivotement (42) supérieur correspondant, la roue dentée (74) étant disposée de façon supportée en rotation sur l'élément de raccordement (44), en particulier **en ce que** les deux guides (38) sont espacés et raccordés de cette façon aux logements (16) respectifs.

10. Dispositif selon l'une des deux revendications précédentes, **caractérisé en ce que** le raccordement bloqué en rotation entre le logement (16) et le segment de denture (68) côté logement et/ou entre le guide (38) et le segment de denture (66) côté guide peut être débloqué temporairement de telle sorte que la position angulaire entre le logement (16) et le segment de denture (68) côté logement ou respectivement entre le guide (38) et le segment de denture (66) côté guide peut pivoter librement.

11. Dispositif selon l'une des revendications précédentes, **caractérisé en ce qu'**il est constitué au moins un logement (16) avec deux branches (80) déformables de façon souple s'étendant le long d'une direction circonférentielle (U) et longitudinale (L) du dispositif de prolongation (12) qui peuvent être posées de l'extérieur sur la circonférence extérieure du dispositif de prolongation (12), les branches (80) présentant chacune une paroi extérieure (82) et une paroi intérieure (84) qui sont raccordées l'une à l'autre en au moins un emplacement (86) de telle sorte que, lors de la traction l'une vers l'autre de la paroi extérieure (82) et de la paroi intérieure (84) dans la direction radiale (R) du dispositif de prolongation (12), il est produit, à un emplacement d'application de la traction (88), au moyen d'un mécanisme d'application de traction (90), une contrainte de pression dans la paroi extérieure (82) agissant dans la direction circonférentielle (U) du dispositif de prolongation (12) en partant de l'emplacement d'application de la traction (88), et une contrainte de traction dans la paroi intérieure (84) agissant dans la direction circonférentielle (U) opposée, ce qui fait que la branche (80) respective est poussée de force contre la circonférence extérieure du dispositif de prolongation (12), et de ce fait le logement (16) peut être disposé de façon coincée sur le dispositif de prolongation (12).

12. Système comprenant un dispositif (10) selon l'une des revendications 1 à 11, ainsi que deux dispositifs de prolongation (12) qui sont logés dans les logements (16), et en particulier deux ancrages osseux (14) avec chacun une tête de fourche, le dispositif (10) étant constitué de telle sorte que les axes de pivotement (26) inférieurs se trouvent dans une zone des dispositifs de prolongation (12) dans laquelle les dispositifs de prolongation (12) entrent en contact avec les ancrages osseux (14) quand ils sont posés sur les ancrages osseux (14), ou de telle sorte que les axes de pivotement (26) inférieurs s'étendent respectivement à travers les ancrages osseux (14) quand les dispositifs de prolongation (12) avec les ancrages osseux (14) qui sont posés dessus sont logés dans le dispositif (10), en particulier les axes de pivotement (26) inférieurs passant à travers la tête de fourche.

13. Système, en particulier selon la revendication 12, comprenant un dispositif (10) selon l'une des revendications 1 à 11 ainsi que deux dispositifs de prolongation (12) qui sont logés dans les logements (16), les dispositifs de prolongation (12) étant, à leur extrémité inférieure, en particulier dans la zone des axes de pivotement (26) inférieurs, espacés du dispositif (10) et n'étant pas dans cette zone en contact avec le dispositif (10).
